# EUROPEAN PATENT SPECIFICATION

(11) **EP 3 515 418 B1**
(45) Date of publication and mention of the grant of the patent: **14.04.2021**
(21) Application number: 17777211.8
(22) Date of filing: 21.09.2017
(51) Int. Cl.: A61K 31/4985, A61K 31/00, A61P 35/00, G01N 33/48, C07K 16/28, A61K 31/5377, A61K 31/53, A61K 31/4745, A61K 31/47, A61K 31/4545, G01N 33/574, C12Q 1/6886

(54) **USE OF C-MET INHIBITORS TO TREAT CANCERS HARBOURING MET MUTATIONS**
VERWENDUNG VON C-MET-HEMMERN ZUR BEHANDLUNG VON KREBS MIT MET-MUTATIONEN
UTILISATION D'INHIBITEURS DE C-MET POUR TRAITER DES CANCERS HÉBERGEANT DES MUTATIONS DE MET

(30) Priority: 22.09.2016 GB 201616116
(43) Date of publication of application: 31.07.2019
(73) Proprietor: Astrazeneca AB, 151 85 Södertälje (SE)
(72) Inventor: FRIGAULT, Melanie Mae, Waltham, MA 02451 (US)
(74) Representative: AstraZeneca
(86) International application number: PCT/EP2017/073894
(87) International publication number: WO 2018/055029

(56) References cited:
- WO-A2-2007/066187
- MARK N. STEIN ET AL: "Response to Crizotinib in a Patient with MET-mutant Papillary Renal Cell Cancer After Progression on Tivantinib", EUROPEAN UROLOGY, vol. 67, no. 2, 1 February 2015 (2015-02-01), pages 353-354, XP055422509, AMSTERDAM, NL ISSN: 0302-2838, DOI: 10.1016/j.eururo.2014.10.012
- M. MEDOVA ET AL: "The Novel ATP-Competitive Inhibitor of the MET Hepatocyte Growth Factor Receptor EMD1214063 Displays Inhibitory Activity against Selected MET-Mutated Variants", MOLECULAR CANCER THERAPEUTICS, vol. 12, no. 11, 23 September 2013 (2013-09-23), pages 2415-2424, XP055163340, ISSN: 1535-7163, DOI: 10.1158/1535-7163.MCT-13-0151
- YANNI ZHANG ET AL: "Biomarker development in MET-targeted therapy", ONCOTARGET, vol. 7, no. 24, 14 June 2016 (2016-06-14), pages 37370-37389, XP055423309, DOI: 10.18632/oncotarget.8276
- A. G. SCHULLER ET AL: "The MET Inhibitor AZD6094 (Savolitinib, HMPL-504) Induces Regression in Papillary Renal Cell Carcinoma Patient-Derived Xenograft Models", CLINICAL CANCER RESEARCH, vol. 21, no. 12, 15 June 2015 (2015-06-15) , pages 2811-2819, XP055422524, US ISSN: 1078-0432, DOI: 10.1158/1078-0432.CCR-14-2685
- PAUL R. GAVINE ET AL: "Volitinib, a potent and highly selective c-Met inhibitor, effectively blocks c-Met signaling and growth in c-MET amplified gastric cancer patient-derived tumor xenograft models", MOLECULAR ONCOLOGY, vol. 9, no. 1, 10 September 2014 (2014-09-10), pages 323-333, XP055422632, AMSTERDAM, NL ISSN: 1574-7891, DOI: 10.1016/j.molonc.2014.08.015
- TONI K. CHOUEIRI ET AL: "Biomarker-Based Phase II Trial of Savolitinib in Patients With Advanced Papillary Renal Cell Cancer", JOURNAL OF CLINICAL ONCOLOGY, vol. 35, no. 26, 10 September 2017 (2017-09-10), pages 2993-3001, XP055423315, US ISSN: 0732-183X, DOI: 10.1200/JCO.2017.72.2967
- M.M. FRIGAULT ET AL: "Response to savolitinib (AZD6094/HMPL-504, a potent and selective MET inhibitor) in a papillary renal cell carcinoma patient harbouring a novel MET activating mutation", ANNALS OF ONCOLOGY., vol. 27, no. suppl_6, 1 October 2016 (2016-10-01), XP055422668, NL ISSN: 0923-7534, DOI: 10.1093/annonc/mdw392.30

<110> AstraZeneca AB
<120> Use of c-Met Inhibitors to Treat Cancers Harbouring MET Mutations
<130> 200560-WO-PCT
<160> 8
<170> PatentIn version 3.5
<210> 1
   <211> 1390
   <212> PRT
   <213> Homo Sapiens
<400> 1
   Met Lys Ala Pro Ala Val Leu Ala Pro Gly Ile Leu Val Leu Leu Phe
   1               5                   10                  15
   Thr Leu Val Gln Arg Ser Asn Gly Glu Cys Lys Glu Ala Leu Ala Lys
               20                  25                  30
   Ser Glu Met Asn Val Asn Met Lys Tyr Gln Leu Pro Asn Phe Thr Ala
           35                  40                  45
   Glu Thr Pro Ile Gln Asn Val Ile Leu His Glu His His Ile Phe Leu
       50                  55                  60
   Gly Ala Thr Asn Tyr Ile Tyr Val Leu Asn Glu Glu Asp Leu Gln Lys
   65                  70                  75                  80
   Val Ala Glu Tyr Lys Thr Gly Pro Val Leu Glu His Pro Asp Cys Phe
                   85                  90                  95
   Pro Cys Gln Asp Cys Ser Ser Lys Ala Asn Leu Ser Gly Gly Val Trp
               100                 105                 110
   Lys Asp Asn Ile Asn Met Ala Leu Val Val Asp Thr Tyr Tyr Asp Asp
           115                 120                 125
   Gln Leu Ile Ser Cys Gly Ser Val Asn Arg Gly Thr Cys Gln Arg His
       130                 135                 140
   Val Phe Pro His Asn His Thr Ala Asp Ile Gln Ser Glu Val His Cys
   145                 150                 155                 160
   Ile Phe Ser Pro Gln Ile Glu Glu Pro Ser Gln Cys Pro Asp Cys Val
                   165                 170                 175
   Val Ser Ala Leu Gly Ala Lys Val Leu Ser Ser Val Lys Asp Arg Phe
               180                 185                 190
   Ile Asn Phe Phe Val Gly Asn Thr Ile Asn Ser Ser Tyr Phe Pro Asp
   195 200 205
   His Pro Leu His Ser Ile Ser Val Arg Arg Leu Lys Glu Thr Lys Asp
   210 215 220
   Gly Phe Met Phe Leu Thr Asp Gln Ser Tyr Ile Asp Val Leu Pro Glu
   225 230 235 240
   Phe Arg Asp Ser Tyr Pro Ile Lys Tyr Val His Ala Phe Glu Ser Asn
   245 250 255
   Asn Phe Ile Tyr Phe Leu Thr Val Gln Arg Glu Thr Leu Asp Ala Gln
   260 265 270
   Thr Phe His Thr Arg Ile Ile Arg Phe Cys Ser Ile Asn Ser Gly Leu
   275 280 285
   His Ser Tyr Met Glu Met Pro Leu Glu Cys Ile Leu Thr Glu Lys Arg
   290 295 300
   Lys Lys Arg Ser Thr Lys Lys Glu Val Phe Asn Ile Leu Gln Ala Ala
   305 310 315 320
   Tyr Val Ser Lys Pro Gly Ala Gln Leu Ala Arg Gln Ile Gly Ala Ser
   325 330 335
   Leu Asn Asp Asp Ile Leu Phe Gly Val Phe Ala Gln Ser Lys Pro Asp
   340 345 350
   Ser Ala Glu Pro Met Asp Arg Ser Ala Met Cys Ala Phe Pro Ile Lys
   355 360 365
   Tyr Val Asn Asp Phe Phe Asn Lys Ile Val Asn Lys Asn Asn Val Arg
   370 375 380
   Cys Leu Gln His Phe Tyr Gly Pro Asn His Glu His Cys Phe Asn Arg
   385 390 395 400
   Thr Leu Leu Arg Asn Ser Ser Gly Cys Glu Ala Arg Arg Asp Glu Tyr
   405 410 415
   Arg Thr Glu Phe Thr Thr Ala Leu Gln Arg Val Asp Leu Phe Met Gly
   420 425 430
   Gln Phe Ser Glu Val Leu Leu Thr Ser Ile Ser Thr Phe Ile Lys Gly
   435 440 445
   Asp Leu Thr Ile Ala Asn Leu Gly Thr Ser Glu Gly Arg Phe Met Gln
   450 455 460
   Val Val Val Ser Arg Ser Gly Pro Ser Thr Pro His Val Asn Phe Leu
   465 470 475 480
   Leu Asp Ser His Pro Val Ser Pro Glu Val Ile Val Glu His Thr Leu
   485 490 495
   Asn Gln Asn Gly Tyr Thr Leu Val Ile Thr Gly Lys Lys Ile Thr Lys
   500 505 510
   Ile Pro Leu Asn Gly Leu Gly Cys Arg His Phe Gln Ser Cys Ser Gln
   515 520 525
   Cys Leu Ser Ala Pro Pro Phe Val Gln Cys Gly Trp Cys His Asp Lys
   530 535 540
   Cys Val Arg Ser Glu Glu Cys Leu Ser Gly Thr Trp Thr Gln Gln Ile
   545 550 555 560
   Cys Leu Pro Ala Ile Tyr Lys Val Phe Pro Asn Ser Ala Pro Leu Glu
   565 570 575
   Gly Gly Thr Arg Leu Thr Ile Cys Gly Trp Asp Phe Gly Phe Arg Arg
   580 585 590
   Asn Asn Lys Phe Asp Leu Lys Lys Thr Arg Val Leu Leu Gly Asn Glu
   595 600 605
   Ser Cys Thr Leu Thr Leu Ser Glu Ser Thr Met Asn Thr Leu Lys Cys
   610 615 620
   Thr Val Gly Pro Ala Met Asn Lys His Phe Asn Met Ser Ile Ile Ile
   625 630 635 640
   Ser Asn Gly His Gly Thr Thr Gln Tyr Ser Thr Phe Ser Tyr Val Asp
   645 650 655
   Pro Val Ile Thr Ser Ile Ser Pro Lys Tyr Gly Pro Met Ala Gly Gly
   660 665 670
   Thr Leu Leu Thr Leu Thr Gly Asn Tyr Leu Asn Ser Gly Asn Ser Arg
   675 680 685
   His Ile Ser Ile Gly Gly Lys Thr Cys Thr Leu Lys Ser Val Ser Asn
   690 695 700
   Ser Ile Leu Glu Cys Tyr Thr Pro Ala Gln Thr Ile Ser Thr Glu Phe
   705 710 715 720
   Ala Val Lys Leu Lys Ile Asp Leu Ala Asn Arg Glu Thr Ser Ile Phe
   725 730 735
   Ser Tyr Arg Glu Asp Pro Ile Val Tyr Glu Ile His Pro Thr Lys Ser
   740 745 750
   Phe Ile Ser Gly Gly Ser Thr Ile Thr Gly Val Gly Lys Asn Leu Asn
   755 760 765
   Ser Val Ser Val Pro Arg Met Val Ile Asn Val His Glu Ala Gly Arg
   770 775 780
   Asn Phe Thr Val Ala Cys Gln His Arg Ser Asn Ser Glu Ile Ile Cys
   785 790 795 800
   Cys Thr Thr Pro Ser Leu Gln Gln Leu Asn Leu Gln Leu Pro Leu Lys
   805 810 815
   Thr Lys Ala Phe Phe Met Leu Asp Gly Ile Leu Ser Lys Tyr Phe Asp
   820 825 830
   Leu Ile Tyr Val His Asn Pro Val Phe Lys Pro Phe Glu Lys Pro Val
   835 840 845
   Met Ile Ser Met Gly Asn Glu Asn Val Leu Glu Ile Lys Gly Asn Asp
   850 855 860
   Ile Asp Pro Glu Ala Val Lys Gly Glu Val Leu Lys Val Gly Asn Lys
   865 870 875 880
   Ser Cys Glu Asn Ile His Leu His Ser Glu Ala Val Leu Cys Thr Val
   885 890 895
   Pro Asn Asp Leu Leu Lys Leu Asn Ser Glu Leu Asn Ile Glu Trp Lys
   900 905 910
   Gln Ala Ile Ser Ser Thr Val Leu Gly Lys Val Ile Val Gln Pro Asp
   915 920 925
   Gln Asn Phe Thr Gly Leu Ile Ala Gly Val Val Ser Ile Ser Thr Ala
   930 935 940
   Leu Leu Leu Leu Leu Gly Phe Phe Leu Trp Leu Lys Lys Arg Lys Gln
   945                 950                 955                 960
   Ile Lys Asp Leu Gly Ser Glu Leu Val Arg Tyr Asp Ala Arg Val His
                   965                 970                 975
   Thr Pro His Leu Asp Arg Leu Val Ser Ala Arg Ser Val Ser Pro Thr
               980                 985                 990
   Thr Glu Met Val Ser Asn Glu Ser  Val Asp Tyr Arg Ala  Thr Phe Pro
           995                 1000                 1005
   Glu Asp  Gln Phe Pro Asn Ser  Ser Gln Asn Gly Ser  Cys Arg Gln
       1010                 1015                 1020
   Val Gln  Tyr Pro Leu Thr Asp  Met Ser Pro Ile Leu  Thr Ser Gly
       1025                 1030                 1035
   Asp Ser  Asp Ile Ser Ser Pro  Leu Leu Gln Asn Thr  Val His Ile
       1040                 1045                 1050
   Asp Leu  Ser Ala Leu Asn Pro  Glu Leu Val Gln Ala  Val Gln His
       1055                 1060                 1065
   Val Val  Ile Gly Pro Ser Ser  Leu Ile Val His Phe  Asn Glu Val
       1070                 1075                 1080
   Ile Gly  Arg Gly His Phe Gly  Cys Val Tyr His Gly  Thr Leu Leu
       1085                 1090                 1095
   Asp Asn  Asp Gly Lys Lys Ile  His Cys Ala Val Lys  Ser Leu Asn
       1100                 1105                 1110
   Arg Ile  Thr Asp Ile Gly Glu  Val Ser Gln Phe Leu  Thr Glu Gly
       1115                 1120                 1125
   Ile Ile  Met Lys Asp Phe Ser  His Pro Asn Val Leu  Ser Leu Leu
       1130                 1135                 1140
   Gly Ile  Cys Leu Arg Ser Glu  Gly Ser Pro Leu Val  Val Leu Pro
       1145                 1150                 1155
   Tyr Met  Lys His Gly Asp Leu  Arg Asn Phe Ile Arg  Asn Glu Thr
       1160                 1165                 1170
   His Asn  Pro Thr Val Lys Asp  Leu Ile Gly Phe Gly  Leu Gln Val
       1175                 1180                 1185
   Ala Lys Gly Met Lys Tyr Leu Ala Ser Lys Lys Phe Val His Arg
       1190                1195                1200
   Asp Leu Ala Ala Arg Asn Cys Met Leu Asp Glu Lys Phe Thr Val
       1205                1210                1215
   Lys Val Ala Asp Phe Gly Leu Ala Arg Asp Met Tyr Asp Lys Glu
       1220                1225                1230
   Tyr Tyr Ser Val His Asn Lys Thr Gly Ala Lys Leu Pro Val Lys
       1235                1240                1245
   Trp Met Ala Leu Glu Ser Leu Gln Thr Gln Lys Phe Thr Thr Lys
       1250                1255                1260
   Ser Asp Val Trp Ser Phe Gly Val Leu Leu Trp Glu Leu Met Thr
       1265                1270                1275
   Arg Gly Ala Pro Pro Tyr Pro Asp Val Asn Thr Phe Asp Ile Thr
       1280                1285                1290
   Val Tyr Leu Leu Gln Gly Arg Arg Leu Leu Gln Pro Glu Tyr Cys
       1295                1300                1305
   Pro Asp Pro Leu Tyr Glu Val Met Leu Lys Cys Trp His Pro Lys
       1310                1315                1320
   Ala Glu Met Arg Pro Ser Phe Ser Glu Leu Val Ser Arg Ile Ser
       1325                1330                1335
   Ala Ile Phe Ser Thr Phe Ile Gly Glu His Tyr Val His Val Asn
       1340                1345                1350
   Ala Thr Tyr Val Asn Val Lys Cys Val Ala Pro Tyr Pro Ser Leu
       1355                1360                1365
   Leu Ser Ser Glu Asp Asn Ala Asp Asp Glu Val Asp Thr Arg Pro
       1370                1375                1380
   Ala Ser Phe Trp Glu Thr Ser
       1385                1390
<210> 2
   <211> 6656
   <212> DNA
   <213> Homo Sapiens
<400> 2
   gcaggtgacc cggaggccct cgccgcccgc ggcgccccga gcgctttgtg agcagatgcg 60
   gagccgagtg gagggcgcga gccagatgcg gggcgacagc tgacttgctg agaggaggcg 120
   gggaggcgcg gagcgcgcgt gtggtccttg cgccgctgac ttctccactg gttcctgggc 180
   accgaaagat aaacctctca taatgaaggc ccccgctgtg cttgcacctg gcatcctcgt 240
   gctcctgttt accttggtgc agaggagcaa tggggagtgt aaagaggcac tagcaaagtc 300
   cgagatgaat gtgaatatga agtatcagct tcccaacttc accgcggaaa cacccatcca 360
   gaatgtcatt ctacatgagc atcacatttt ccttggtgcc actaactaca tttatgtttt 420
   aaatgaggaa gaccttcaga aggttgctga gtacaagact gggcctgtgc tggaacaccc 480
   agattgtttc ccatgtcagg actgcagcag caaagccaat ttatcaggag gtgtttggaa 540
   agataacatc aacatggctc tagttgtcga cacctactat gatgatcaac tcattagctg 600
   tggcagcgtc aacagaggga cctgccagcg acatgtcttt ccccacaatc atactgctga 660
   catacagtcg gaggttcact gcatattctc cccacagata gaagagccca gccagtgtcc 720
   tgactgtgtg gtgagcgccc tgggagccaa agtcctttca tctgtaaagg accggttcat 780
   caacttcttt gtaggcaata ccataaattc ttcttatttc ccagatcatc cattgcattc 840
   gatatcagtg agaaggctaa aggaaacgaa agatggtttt atgtttttga cggaccagtc 900
   ctacattgat gttttacctg agttcagaga ttcttacccc attaagtatg tccatgcctt 960
   tgaaagcaac aattttattt acttcttgac ggtccaaagg gaaactctag atgctcagac 1020
   ttttcacaca agaataatca ggttctgttc cataaactct ggattgcatt cctacatgga 1080
   aatgcctctg gagtgtattc tcacagaaaa gagaaaaaag agatccacaa agaaggaagt 1140
   gtttaatata cttcaggctg cgtatgtcag caagcctggg gcccagcttg ctagacaaat 1200
   aggagccagc ctgaatgatg acattctttt cggggtgttc gcacaaagca agccagattc 1260
   tgccgaacca atggatcgat ctgccatgtg tgcattccct atcaaatatg tcaacgactt 1320
   cttcaacaag atcgtcaaca aaaacaatgt gagatgtctc cagcattttt acggacccaa 1380
   tcatgagcac tgctttaata ggacacttct gagaaattca tcaggctgtg aagcgcgccg 1440
   tgatgaatat cgaacagagt ttaccacagc tttgcagcgc gttgacttat tcatgggtca 1500
   attcagcgaa gtcctcttaa catctatatc caccttcatt aaaggagacc tcaccatagc 1560
   taatcttggg acatcagagg gtcgcttcat gcaggttgtg gtttctcgat caggaccatc 1620
   aacccctcat gtgaattttc tcctggactc ccatccagtg tctccagaag tgattgtgga 1680
   gcatacatta aaccaaaatg gctacacact ggttatcact gggaagaaga tcacgaagat 1740
   cccattgaat ggcttgggct gcagacattt ccagtcctgc agtcaatgcc tctctgcccc 1800
   accctttgtt cagtgtggct ggtgccacga caaatgtgtg cgatcggagg aatgcctgag 1860
   cgggacatgg actcaacaga tctgtctgcc tgcaatctac aaggttttcc caaatagtgc     1920
   accccttgaa ggagggacaa ggctgaccat atgtggctgg gactttggat ttcggaggaa 1980
   taataaattt gatttaaaga aaactagagt tctccttgga aatgagagct gcaccttgac     2040
   tttaagtgag agcacgatga atacattgaa atgcacagtt ggtcctgcca tgaataagca     2100
   tttcaatatg tccataatta tttcaaatgg ccacgggaca acacaataca gtacattctc     2160
   ctatgtggat cctgtaataa caagtatttc gccgaaatac ggtcctatgg ctggtggcac     2220
   tttacttact ttaactggaa attacctaaa cagtgggaat tctagacaca tttcaattgg     2280
   tggaaaaaca tgtactttaa aaagtgtgtc aaacagtatt cttgaatgtt ataccccagc     2340
   ccaaaccatt tcaactgagt ttgctgttaa attgaaaatt gacttagcca accgagagac     2400
   aagcatcttc agttaccgtg aagatcccat tgtctatgaa attcatccaa ccaaatcttt     2460
   tattagtggt gggagcacaa taacaggtgt tgggaaaaac ctgaattcag ttagtgtccc     2520
   gagaatggtc ataaatgtgc atgaagcagg aaggaacttt acagtggcat gtcaacatcg     2580
   ctctaattca gagataatct gttgtaccac tccttccctg caacagctga atctgcaact     2640
   ccccctgaaa accaaagcct ttttcatgtt agatgggatc ctttccaaat actttgatct     2700
   catttatgta cataatcctg tgtttaagcc ttttgaaaag ccagtgatga tctcaatggg     2760
   caatgaaaat gtactggaaa ttaagggaaa tgatattgac cctgaagcag ttaaaggtga     2820
   agtgttaaaa gttggaaata agagctgtga gaatatacac ttacattctg aagccgtttt     2880
   atgcacggtc cccaatgacc tgctgaaatt gaacagcgag ctaaatatag agtggaagca     2940
   agcaatttct tcaaccgtcc ttggaaaagt aatagttcaa ccagatcaga atttcacagg     3000
   attgattgct ggtgttgtct caatatcaac agcactgtta ttactacttg ggtttttcct     3060
   gtggctgaaa aagagaaagc aaattaaaga tctgggcagt gaattagttc gctacgatgc     3120
   aagagtacac actcctcatt tggataggct tgtaagtgcc cgaagtgtaa gcccaactac     3180
   agaaatggtt tcaaatgaat ctgtagacta ccgagctact tttccagaag atcagtttcc     3240
   taattcatct cagaacggtt catgccgaca agtgcagtat cctctgacag acatgtcccc     3300
   catcctaact agtggggact ctgatatatc cagtccatta ctgcaaaata ctgtccacat     3360
   tgacctcagt gctctaaatc cagagctggt ccaggcagtg cagcatgtag tgattgggcc     3420
   cagtagcctg attgtgcatt tcaatgaagt cataggaaga gggcattttg gttgtgtata     3480
   tcatgggact ttgttggaca atgatggcaa gaaaattcac tgtgctgtga aatccttgaa     3540
   cagaatcact gacataggag aagtttccca atttctgacc gagggaatca tcatgaaaga     3600
   ttttagtcat cccaatgtcc tctcgctcct gggaatctgc ctgcgaagtg aagggtctcc     3660
   gctggtggtc ctaccataca tgaaacatgg agatcttcga aatttcattc gaaatgagac     3720
   tcataatcca actgtaaaag atcttattgg ctttggtctt caagtagcca aaggcatgaa     3780
   atatcttgca agcaaaaagt ttgtccacag agacttggct gcaagaaact gtatgctgga 3840
   tgaaaaattc acagtcaagg ttgctgattt tggtcttgcc agagacatgt atgataaaga 3900
   atactatagt gtacacaaca aaacaggtgc aaagctgcca gtgaagtgga tggctttgga 3960
   aagtctgcaa actcaaaagt ttaccaccaa gtcagatgtg tggtcctttg gcgtgctcct 4020
   ctgggagctg atgacaagag gagccccacc ttatcctgac gtaaacacct ttgatataac 4080
   tgtttacttg ttgcaaggga gaagactcct acaacccgaa tactgcccag accccttata 4140
   tgaagtaatg ctaaaatgct ggcaccctaa agccgaaatg cgcccatcct tttctgaact 4200
   ggtgtcccgg atatcagcga tcttctctac tttcattggg gagcactatg tccatgtgaa 4260
   cgctacttat gtgaacgtaa aatgtgtcgc tccgtatcct tctctgttgt catcagaaga 4320
   taacgctgat gatgaggtgg acacacgacc agcctccttc tgggagacat catagtgcta 4380
   gtactatgtc aaagcaacag tccacacttt gtccaatggt tttttcactg cctgaccttt 4440
   aaaaggccat cgatattctt tgctcttgcc aaaattgcac tattatagga cttgtattgt 4500
   tatttaaatt actggattct aaggaatttc ttatctgaca gagcatcaga accagaggct 4560
   tggtcccaca ggccacggac caatggcctg cagccgtgac aacactcctg tcatattgga 4620
   gtccaaaact tgaattctgg gttgaatttt ttaaaaatca ggtaccactt gatttcatat 4680
   gggaaattga agcaggaaat attgagggct tcttgatcac agaaaactca gaagagatag 4740
   taatgctcag gacaggagcg gcagccccag aacaggccac tcatttagaa ttctagtgtt 4800
   tcaaaacact tttgtgtgtt gtatggtcaa taacattttt cattactgat ggtgtcattc 4860
   acccattagg taaacattcc cttttaaatg tttgtttgtt ttttgagaca ggatctcact 4920
   ctgttgccag ggctgtagtg cagtggtgtg atcatagctc actgcaacct ccacctccca 4980
   ggctcaagcc tcccgaatag ctgggactac aggcgcacac caccatcccc ggctaatttt 5040
   tgtatttttt gtagagacgg ggttttgcca tgttgccaag gctggtttca aactcctgga 5100
   ctcaagaaat ccacccacct cagcctccca aagtgctagg attacaggca tgagccactg 5160
   cgcccagccc ttataaattt ttgtatagac attcctttgg ttggaagaat atttataggc 5220
   aatacagtca aagtttcaaa atagcatcac acaaaacatg tttataaatg aacaggatgt 5280
   aatgtacata gatgacatta agaaaatttg tatgaaataa tttagtcatc atgaaatatt 5340
   tagttgtcat ataaaaaccc actgtttgag aatgatgcta ctctgatcta atgaatgtga 5400
   acatgtagat gttttgtgtg tattttttta aatgaaaact caaaataaga caagtaattt 5460
   gttgataaat atttttaaag ataactcagc atgtttgtaa agcaggatac attttactaa 5520
   aaggttcatt ggttccaatc acagctcata ggtagagcaa agaaagggtg gatggattga 5580
   aaagattagc ctctgtctcg gtggcaggtt cccacctcgc aagcaattgg aaacaaaact 5640
   tttggggagt tttattttgc attagggtgt gttttatgtt aagcaaaaca tactttagaa 5700
   acaaatgaaa aaggcaattg aaaatcccag ctatttcacc tagatggaat agccaccctg 5760
   agcagaactt tgtgatgctt cattctgtgg aattttgtgc ttgctactgt atagtgcatg 5820
   tggtgtaggt tactctaact ggttttgtcg acgtaaacat ttaaagtgtt atatttttta 5880
   taaaaatgtt tatttttaat gatatgagaa aaattttgtt aggccacaaa aacactgcac 5940
   tgtgaacatt ttagaaaagg tatgtcagac tgggattaat gacagcatga ttttcaatga 6000
   ctgtaaattg cgataaggaa atgtactgat tgccaataca ccccaccctc attacatcat 6060
   caggacttga agccaagggt taacccagca agctacaaag agggtgtgtc acactgaaac 6120
   tcaatagttg agtttggctg ttgttgcagg aaaatgatta taactaaaag ctctctgata 6180
   gtgcagagac ttaccagaag acacaaggaa ttgtactgaa gagctattac aatccaaata 6240
   ttgccgtttc ataaatgtaa taagtaatac taattcacag agtattgtaa atggtggatg 6300
   acaaaagaaa atctgctctg tggaaagaaa gaactgtctc taccagggtc aagagcatga 6360
   acgcatcaat agaaagaact cggggaaaca tcccatcaac aggactacac acttgtatat 6420
   acattcttga gaacactgca atgtgaaaat cacgtttgct atttataaac ttgtccttag 6480
   attaatgtgt ctggacagat tgtgggagta agtgattctt ctaagaatta gatacttgtc 6540
   actgcctata cctgcagctg aactgaatgg tacttcgtat gttaatagtt gttctgataa 6600
   atcatgcaat taaagtaaag tgatgcaaca tcttgtaaaa aaaaaaaaaa aaaaaa 6656
<210> 3
   <211> 19
   <212> DNA
   <213> Homo sapiens
<400> 3
   agatacgacg cccgggtgc 19
<210> 4
   <211> 18
   <212> DNA
   <213> Homo Sapiens
<400> 4
   agaaaatcca ctgcgccg 18
<210> 5
   <211> 18
   <212> DNA
   <213> Homo Sapiens
<400> 5
   cgtgcacaac aagacagg 18
<210> 6
   <211> 18
   <212> DNA
   <213> Homo sapiens
<400> 6
   tctggtcatc agctccca 18
<210> 7
   <211> 19
   <212> DNA
   <213> Homo sapiens
<400> 7
   agcaggctca gcacgttgg 19
<210> 8
   <211> 19
   <212> DNA
   <213> Homo sapiens
<400> 8
   aactggtcct cggggaagg 19

## Description

### FIELD

This specification relates to c-Met receptor tyrosine kinase ("c-Met") inhibitors and their use in the treatment of cancers characterised by certain MET mutations (e.g. cancers comprising cells that express a MET protein having a MET V1092I, MET H1094L or MET L1195F mutation). This specification further relates to the use of MET mutation status to select patients suitable for treatment with a c-Met inhibitor, and to methods of treating cancers characterised by certain MET mutations with a c-Met inhibitor.

### BACKGROUND

The MET protein, also known as c-Met receptor tyrosine kinase, is a transmembrane receptor essential for embryonic development and wound healing. The MET receptor is normally activated through interaction with its specific ligand, hepatocyte growth factor (HGF), and is the only high-affinity cell surface receptor for HGF (Bottaro *et al.* 1991). The MET receptor is deregulated in many types of human malignancies, including cancers of the kidney, liver, stomach, lung, breast, and brain. Aberrant activation of the HGF/c-Met axis in tumours triggers tumour growth, promotes tumour angiogenesis, and induces tumour metastasis. In addition, abnormal MET activation is associated with drug resistance and is correlated with poor prognosis. Recently, inhibition of the c-Met signalling pathway has become an area of interest in the search for potential new therapies for cancers driven by c-Met activation.

Germline mutations of the MET gene locus 7q31 have been detected in patients with hereditary papillary renal cell carcinoma (PRCC) and in sporadic PRCC (Salvi *et al.* **2008,** Schmidt *et al.* **1997,** Schmidt *et al.* **1999).** It is commonly believed that Type I tumours are generally associated with a MET mutation, whether sporadic or hereditary. Although MET mutations have been associated with Type I PRCC in cohort analyses, MET mutations have also been reported in the non-Type I histological subtype of PRCC (Albiges *et al.* **2014,** Linehan *et al.* **2016).** MET somatic mutations in sporadic PRCC have been reported, while analysis of only Type I PRCC cases show a 21.6% (11/51) frequency of MET kinase domain mutations (Albiges *et al.* **2014),** and a report from The Cancer Genome Atlas (TCGA) indicates 13/75 (17.3%) Type I PRCC and 1/26 (3.8%) unclassified PRCC to harbour somatic mutation in the MET kinase domain (Linehan *et al.* **2015).** All reported MET mutations have been missense mutations, and no nonsense or loss of function mutations have been found. Furthermore, MET mutations have been reported by TCGA as the most frequently mutated gene in PRCC, 17/157 (10.8%).

At present, there is no approved therapy indicated specifically for the treatment of PRCC, and therefore patients with PRCC are treated in a similar manner as clear-cell (known also as conventional) RCC patients (RCC = renal cell carcinoma). Primary treatment of patients with early stage RCC involves radical surgical resection which cures more than 40% to 60% of patients, although many patients with localized disease will relapse (Linehan *et al.* 2001). Approximately 25% of patients will present with locally advanced or metastatic disease at diagnosis. The prognosis for patients with distant metastases is poor, with a 5-year survival rate of 10% in patients with stage IV disease. Approved agents clear cell RCC target the VEGF pathway and include sunitinib, sorafenib, bevacizumab, pazopanib, and axitinib. Agents targeting the mTOR pathway that are approved include temsirolimus and everolimus. Best response rates for PRCC patients with any VEGF or mTOR pathway inhibitors is 11% ORR.

Savolitinib is a potent and selective small molecule c-Met kinase inhibitor (Jia H. et al., J. Med. Chem. 2014; 7577). Savolitinib was found to inhibit c-Met kinase at the enzyme and cell levels with IC₅₀s of 4nM for both enzyme and Met phosphorylation in the cell. Consistent with its potent enzyme and cell activity, savolitinib was found to inhibit cell growth in vitro against tumours with MET gene amplification in the absence of HGF stimulation with IC₅₀s generally below 10nM. In human xenograft models in mouse, savolitinib demonstrated excellent anti-tumour activity against MET gene amplified gastric and lung tumours with ED50s below 5 mg/kg following once-daily oral treatment.

MET is not yet a clinically validated target. However, non-selective inhibitors which incidentally target MET have been approved for use in therapy. These include cabozantinib (RET, FLT3, KIT, MET, VEGFR2), which is approved in the treatment of Medullary Thyroid Cancer (as a result of its RET activity) and clear cell RCC (as a result of its VEGFR2 activity); and crizotinib (ALK, MET) which is approved to treat Non-Small Cell Lung Cancer with ALK fusions. Although these drugs have MET kinase inhibitory activity, patients with MET driven disease are not yet served by targeted therapies. The use of savolitinib, a potent and selective MET inhibitor may provide the clinical validation needed to provide anti-tumour benefit to patients with MET gene amplifications, MET mutations and other MET pathway biomarkers that remain to be validated.

The present specification concerns the characterization of certain MET mutations which may be useful as biomarkers for c-Met therapies, including METL1195F, MET V1092I and MET H1094L mutations. The MET L1195F mutation has been previously reported in papillary renal carcinoma patients (Schmidt et al. Nature Genetics 1997, Albiges et al. CCR 2014), but has not been functionally investigated before. Data reported in this specification shows for the first time that this MET L1195F mutant is actionable. MET L1195F is phosphorylated when overexpressed demonstrating its propensity to activate the MET pathway including ERK1/2 activation. The MET L1195F mutant is demonstrated to be sensitive to savolitinib inhibition and can confer a growth advantage in stable cell lines to a similar extent to a MET mutation that has been pre-clinically functionally characterized M1250T (Bardelli et al. PNAS 1998).

The present specification also provides clinical validation that MET L1195F mutants are actionable, showing that a PRCC patient harbouring a MET L1195F mutation in the absence of any other MET alteration derives benefit from monotherapy with savolitinib. Taken together, the pre-clinical functional characterization of Met L1195F and the clinical signal provide the basis for classifying this kinase domain mutation as the first reported to be functionally understood at the bench and demonstrate clinical anti-tumour validation. Of note, the MET L1195F mutant is found in the context of Type II histological subtype of PRCC patients which further provides a case for a molecular classification of PRCC rather than the classical Type I association with MET gene mutations.

Although MET inhibitors are thought to be ideally developed in MET gene copy number amplification settings (Garber, Nature Reviews Drug Discovery 2014**),** this specification provides evidence for the additional use of MET mutants as actionable biomarkers. As will be seen, the present specification demonstrates that certain MET mutations including METL1195F, MET V1092I and MET H1094L are actionable as biomarkers for c-Met inhibition. Cancer cell MET mutation status may therefore be useful to identify cancer patients (for example papillary renal cell carcinoma patients) more likely to respond to treatment with a c-Met inhibitor (for example savolitinib or a pharmaceutically acceptable salt thereof). This will not be true for all possible MET mutations: the finding that MET L1195F, MET V1092I and MET H1094L are actionable biomarkers for c-Met therapy is particularly surprising given that when examined in the same *in-vitro* tests described herein, cells comprising a MET Y1230H mutation (another c-Met kinase domain mutation previously reported in papillary renal carcinoma patients and predicted to be activating) were not sensitive to treatment with a c-Met inhibitor.

### SUMMARY

The present invention relates to a c-Met inhibitor for use in the treatment of cancer, where the cancer is characterised by a MET 1195 mutation and where the c-Met inhibitor is savolitinib or a pharmaceutically acceptable salt thereof.

The invention also relates to a method of screening cancer patients to establish their suitability for treatment with a c-Met inhibitor, comprising analysing a representative sample of a patient's cancer in-vitro to determine whether the cancer is characterised by a MET 1195 mutation, where if the patient's cancer is found to be characterised by a MET 1195 mutation, the patient is suitable for treatment with the c-Met inhibitor, and where the c-Met inhibitor is savolitinib or a pharmaceutically acceptable salt thereof.

This specification also describes, in part, a c-Met inhibitor for use in the treatment of cancer, where the cancer is characterised by a MET 1092, MET 1094 or MET 1195 mutation.

This specification also describes, in part, a method of screening cancer patients to establish their suitability for treatment with a c-Met inhibitor, comprising analysing a representative sample of a patient's cancer *in-vitro* to determine whether the cancer is characterised by a MET 1092, MET 1094 or MET 1195 mutation, where if the patient's cancer is found to be characterised by a MET 1092, MET 1094 or MET 1195 mutation, the patient is suitable for treatment with a c-Met inhibitor.

This specification also describes, in part, use of a c-Met inhibitor in the preparation of a medicament for the treatment of cancer, where the cancer is characterised by a MET 1092, MET 1094 or MET 1195 mutation.

This specification also describes, in part, a method for treating cancer comprising administering a therapeutically effective amount of a c-Met inhibitor to a patient in need of such treatment, wherein said cancer is characterised by a MET 1092, MET 1094 or MET 1195 mutation.

This specification also describes, in part, a method for treating cancer in a patient in need of such treatment, comprising the steps of: a) requesting a test whose results can determine whether the patient's cancer is characterised by a MET 1092, MET 1094 or MET 1195 mutation; and b) administering a therapeutically effective amount of a c-Met inhibitor to the patient if the patient's cancer is found to be characterised by a MET 1092, MET 1094 or MET 1195 mutation.

### BRIEF DESCRIPTION OF THE FIGURES

**Figure 1****:** Phosphorylation of the MET Receptor Tyrosine Kinase and Effect of Point Mutations on Sensitivity to c-Met Inhibition with Savolitinib
**Figure 2****:** Dose Response of MET Wild-type (WT) to Savolitinib and Other c-Met Inhibitors
**Figure 3****:** Dose Response of a MET M1250T Point Mutant to Savolitinib and Other c-Met Inhibitors
**Figure 4****:** Dose Response of a MET V1092I Point Mutant to Savolitinib and Other c-Met Inhibitors
**Figure 5****:** Dose Response of a MET H1094L Point Mutant to Savolitinib and Other c-Met Inhibitors
**Figure 6****:** Dose Response of a MET L1195F Point Mutant to Savolitinib and Other c-Met Inhibitors
**Figure 7****:** Dose Response of a MET Y1230H Point Mutant to Savolitinib and Other c-Met Inhibitors
**Figure 8****:** Stable Expression of MET Mutant Clones in MET Mutant Cell Lines
**Figure 9****:** Transformation Capacity of Parental Ba/F3 Stable Cell Lines

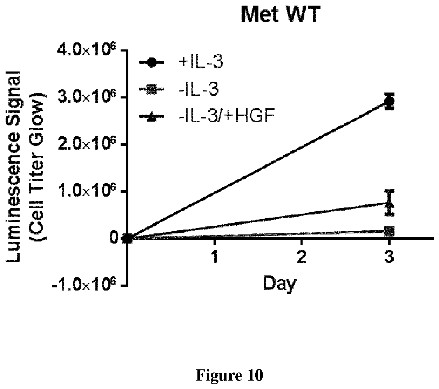

**Figure 10****:** Transformation Capacity of MET WT Stable Cell Lines

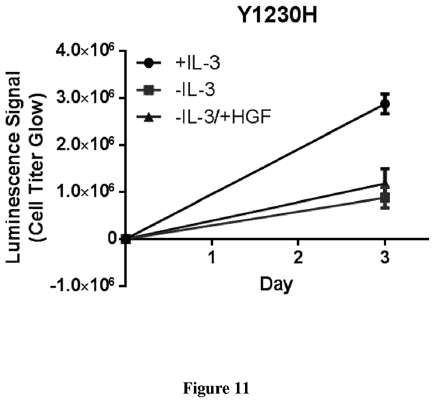

**Figure 11****:** Transformation Capacity of MET Y1230H Point Mutant Stable Cell Lines

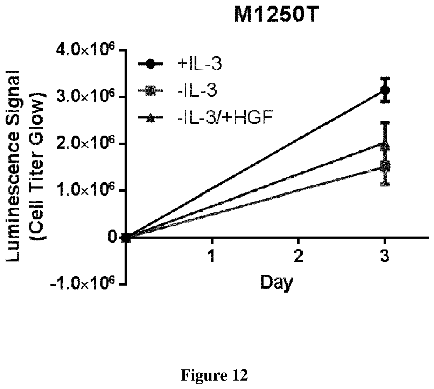

**Figure 12****:** Transformation Capacity of MET M1250T Point Mutant Stable Cell Lines

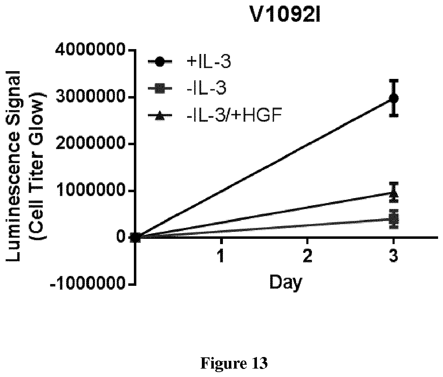

**Figure 13****:** Transformation Capacity of MET V1092I Point Mutant Stable Cell Lines

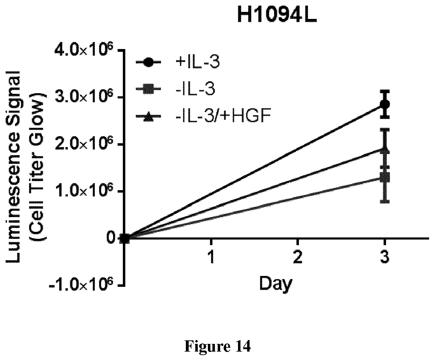

**Figure 14****:** Transformation Capacity of MET H1 094L Point Mutant Stable Cell Lines

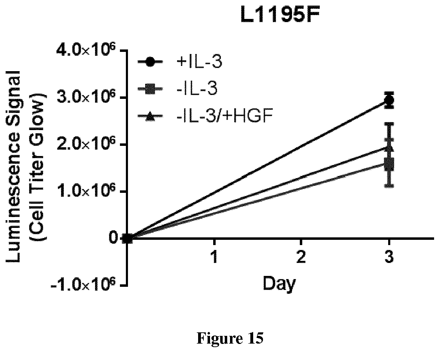

**Figure 15****:** Transformation Capacity of MET L1195F Point Mutant Stable Cell Lines

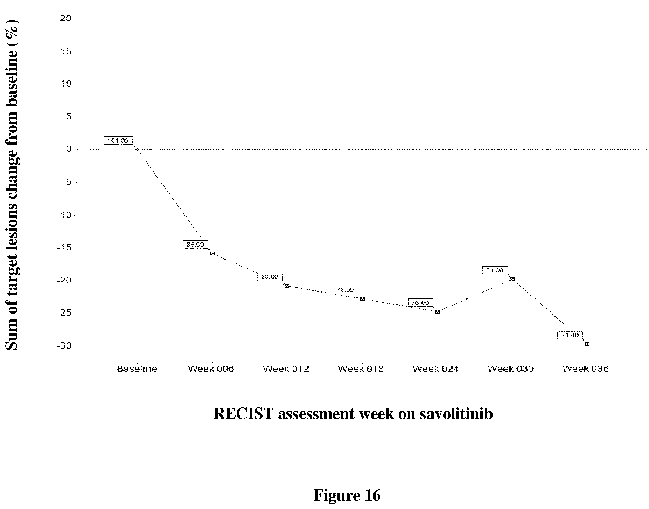

**Figure 16****:** MET L1195F Mutant Papillary Renal Cell Carcinoma Patient Tumour Response

### ILLUSTRATIVE EMBODIMENTS

Many embodiments are detailed in this specification and will be apparent to a reader skilled in the art. The embodiments are not to be considered limiting.

In the first embodiment there is provided a c-Met inhibitor for use in the treatment of cancer, where the cancer is characterised by a MET 1092, MET 1094 or MET 1195 mutation.

A "c-Met inhibitor" is a molecule that reduces the activity of c-Met receptor tyrosine kinase. c-Met inhibitors include both small molecules (whether selective or unselective) and biomolecules (for example antibodies, both natural and engineered), c-Met inhibitors may inhibit c-Met receptor tyrosine kinase either directly (for example by binding directly to the enzyme) or indirectly (for example by binding to hepatocyte growth factor, the natural ligand of c-Met receptor tyrosine kinase).

Example c-Met inhibitors include AMG-208, AMG-337, AMG-458, PHA-665752, SU11274, NPS-1034, SGX-523, BMS-777607, tepotinib, BMS-794833, NVP-BVU972, MK-2461, MGCD-265, golvatinib, JNJ-38877605, BMS-754807, PF-04217903, savolitinib, crizotinib, tivantinib, cabozantinib, foretinib, capmatinib (INC280), onartuzumab, ficlatuzumab or rilotumumab.

In any embodiment where a c-Met inhibitor is mentioned, the c-Met inhibitor may be AMG-208 or a pharmaceutically acceptable salt thereof, AMG-458 or a pharmaceutically acceptable salt thereof, PHA-665752 or a pharmaceutically acceptable salt thereof, SU11274 or a pharmaceutically acceptable salt thereof, NPS-1034 or a pharmaceutically acceptable salt thereof, SGX-523 or a pharmaceutically acceptable salt thereof, BMS-777607 or a pharmaceutically acceptable salt thereof, tepotinib or a pharmaceutically acceptable salt thereof, BMS-794833 or a pharmaceutically acceptable salt thereof, NVP-BVU972 or a pharmaceutically acceptable salt thereof, MK-2461 or a pharmaceutically acceptable salt thereof, MGCD-265 or a pharmaceutically acceptable salt thereof, golvatinib or a pharmaceutically acceptable salt thereof, JNJ-38877605 or a pharmaceutically acceptable salt thereof, BMS-754807 or a pharmaceutically acceptable salt thereof, PF-04217903 or a pharmaceutically acceptable salt thereof, savolitinib or a pharmaceutically acceptable salt thereof, crizotinib or a pharmaceutically acceptable salt thereof, tivantinib or a pharmaceutically acceptable salt thereof, cabozantinib or a pharmaceutically acceptable salt thereof, foretinib or a pharmaceutically acceptable salt thereof, capmatinib or a pharmaceutically acceptable salt thereof, onartuzumab, ficlatuzumab or rilotumumab.

In any embodiment where a c-Met inhibitor is mentioned, the c-Met inhibitor may be savolitinib or a pharmaceutically acceptable salt thereof, crizotinib or a pharmaceutically acceptable salt thereof, tivantinib or a pharmaceutically acceptable salt thereof, cabozantinib or a pharmaceutically acceptable salt thereof, foretinib or a pharmaceutically acceptable salt thereof, capmatinib or a pharmaceutically acceptable salt thereof, onartuzumab, ficlatuzumab or rilotumumab.

In any embodiment where a c-Met inhibitor is mentioned, the c-Met inhibitor may be savolitinib or a pharmaceutically acceptable salt thereof, crizotinib or a pharmaceutically acceptable salt thereof, tivantinib or a pharmaceutically acceptable salt thereof, cabozantinib or a pharmaceutically acceptable salt thereof, foretinib or a pharmaceutically acceptable salt thereof, or capmatinib or a pharmaceutically acceptable salt thereof.

In any embodiment where a c-Met inhibitor is mentioned, the c-Met inhibitor may be savolitinib or a pharmaceutically acceptable salt thereof, crizotinib or a pharmaceutically acceptable salt thereof or capmatinib or a pharmaceutically acceptable salt thereof.

In any embodiment where a c-Met inhibitor is mentioned, the c-Met inhibitor may be capmatinib or a pharmaceutically acceptable salt thereof.

In any embodiment where a c-Met inhibitor is mentioned, the c-Met inhibitor may be crizotinib or a pharmaceutically acceptable salt thereof.

In any embodiment where a c-Met inhibitor is mentioned, the c-Met inhibitor may be savolitinib or a pharmaceutically acceptable salt thereof.

In any embodiment where a c-Met inhibitor is mentioned, the c-Met inhibitor may be onartuzumab, ficlatuzumab or rilotumumab According to the present invention, the c-Met inhibitor is savolitinib or a pharmaceutically acceptable salt thereof.

"Savolitinib" is described in WO2011079804 (as compound 270 on page 106) Savolitinib as the free base has the following structure:

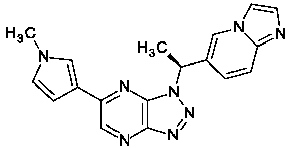

The term "pharmaceutically acceptable" is used to specify that an object (for example a salt or an excipient) is suitable for use in patients. An example list of pharmaceutically acceptable salts can be found in the Handbook of Pharmaceutical Salts: Properties, Selection and Use, 2nd Revised Edition; editors P. H. Stahl and C. G. Wermuth; Wiley 2011; ISBN: 978-3-90639-051-2;

In any embodiment where a pharmaceutically acceptable salt is mentioned, the pharmaceutically acceptable salt may be any such salt found in the Handbook of Pharmaceutical Salts: Properties, Selection and Use, 2nd Revised Edition; editors P. H. Stahl and C. G. Wermuth; Wiley 2011; ISBN: 978-3-90639-051-2.

c-Met inhibitors may exist in optically active or racemic forms by virtue of an asymmetric carbon atom. For example, savolitinib and its pharmaceutically acceptable salts possess such an asymmetric carbon atom. The synthesis of optically active forms may be carried out by standard techniques of organic chemistry well known in the art, for example by synthesis using optically active materials or by resolution of a racemic form.

In any embodiment where a c-Met inhibitor is mentioned, the c-Met inhibitor may comprise a pharmaceutical composition where a single optical isomer is present in an enantiomeric or diastereomeric excess (%ee) of ≥ 95%, ≥ 98% or ≥ 99%.

In any embodiment where a c-Met inhibitor is mentioned, the c-Met inhibitor may comprise a pharmaceutical composition where a single optical isomer is present in an enantiomeric or diastereomeric excess (%ee) of ≥ 99%.

In any embodiment where savolitinib or a pharmaceutically acceptable salt thereof is mentioned, the savolitinib or a pharmaceutically acceptable salt thereof may be an (S)-optical isomer being in an enantiomeric excess (%ee) of ≥ 95%, ≥ 98% or ≥ 99%. In any embodiment where an (S)-isomer of savolitinib or a pharmaceutically acceptable salt thereof is mentioned, the (S)-optical isomer may be present in an enantiomeric excess (%ee) of > 99%.

c-Met inhibitors may be administered as pharmaceutical compositions, comprising one or more pharmaceutically acceptable excipients. The excipient(s) selected for inclusion in a particular composition will depend on factors such as the mode of administration and the form of the composition provided. Suitable pharmaceutically acceptable excipients are well known to persons skilled in the art and are described, for example, in the Handbook of Pharmaceutical Excipients, 6th Edition; editors R. C. Rowe, P.J. Sheskey and M. Quinn; Pharmaceutical Press. Pharmaceutically acceptable excipients may function as, for example, adjuvants, diluents, carriers, stabilisers, flavourings, colorants, fillers, binders, disintegrants, lubricants, glidants, thickening agents and coating agents. Certain pharmaceutically acceptable excipients may serve more than one function and may serve alternative functions depending on how much of the excipient is present in the composition and what other excipients are present in the composition.

Pharmaceutical compositions comprising c-Met inhibitors may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing), or as a suppository for rectal dosing. The compositions may be obtained by conventional procedures well known in the art. Compositions intended for oral use may contain additional components, for example, one or more colouring, sweetening, flavouring and/or preservative agents.

In any embodiment where a c-Met inhibitor is mentioned, the c-Met inhibitor may be administered orally.

In any embodiment where a c-Met inhibitor is mentioned, the c-Met inhibitor may be administered orally as an tablet or capsule.

c-Met inhibitors may be administered in a unit dose form to achieve a therapeutically-effective dose. A unit dose form such as a tablet or capsule will usually contain, for example, 0.1-5000 mg of c-Met inhibitor. The overall dose and dose schedule will necessarily be varied depending upon the host treated, the particular route of administration, any therapies being co-administered, and the severity of the illness being treated. Accordingly the practitioner who is treating any particular patient may determine the optimum dosage with reference to any regulatory label associated with the c-Met inhibitor.

In any embodiment where a c-Met inhibitor or a pharmaceutically acceptable salt thereof is mentioned, the c-Met inhibitor or a pharmaceutically acceptable salt thereof may be administered in a pharmaceutical composition comprising between 0.1-1mg, 1-10 mg, 10-50mg, 50-100mg, 100-500mg, or 500mg to 5000mg of c-Met inhibitor or a pharmaceutically acceptable salt thereof.

In any embodiment where savolitinib or a pharmaceutically acceptable salt thereof is mentioned, the savolitinib or a pharmaceutically acceptable salt thereof may be administered in a pharmaceutical composition comprising between 0.1-1mg, 1-10 mg, 10-50mg, 50-100mg, 100-500mg, or 500mg to 5000mg of savolitinib or a pharmaceutically acceptable salt thereof.

In any embodiment where savolitinib or a pharmaceutically acceptable salt thereof is mentioned, the savolitinib or a pharmaceutically acceptable salt thereof may be administered in a pharmaceutical composition comprising 100-1000 mg of savolitinib in its free base form.

In any embodiment where savolitinib or a pharmaceutically acceptable salt thereof is mentioned, the savolitinib or a pharmaceutically acceptable salt thereof may be administered in a pharmaceutical composition comprising 500-1000 mg of savolitinib in its free base form.

In any embodiment where savolitinib or a pharmaceutically acceptable salt thereof is mentioned, the savolitinib or a pharmaceutically acceptable salt thereof may be administered in a pharmaceutical composition comprising 600 mg of savolitinib in its free base form.

In any embodiment where savolitinib or a pharmaceutically acceptable salt thereof is mentioned, the savolitinib or a pharmaceutically acceptable salt thereof may be administered in a pharmaceutical composition comprising 600 mg of savolitinib in its free base form, dosed once daily.

A cancer that "is characterised by a MET 1092, MET 1094 or MET 1195 mutation" comprises cells which express MET protein comprising a MET 1092, MET 1094 or MET 1195 mutation.

A "MET protein comprising a MET 1092, MET 1094 or MET 1195 mutation" is a MET protein differing from wild-type MET by a mutation or variance (for example a substitution or deletion) occurring at one or more of the specified amino acid positions.

The sequence of the wild-type MET protein is described in RefSeq: NCBI Reference Sequence Database (RefSeq Accession NM_00245), which is provided herein (SEQ ID NO: 1). This sequence is used as the reference sequence to describe the protein mutations described in the present specification. For example, references to a cancer that is characterised by a MET 1092, MET 1094 or MET 1195 mutation describe a cancer comprising cells which express MET protein comprising at least one mutation or variance at one or more of positions 1092, 1094 or 1195 of the sequence recited herein.

MET mutations in the long isoform of MET with 18 additional amino acids would result in the same biological consequence, i.e. sensitivity to c-Met inhibitors.

When it is mentioned that a cancer is characterised by a MET 1092, MET 1094 or MET 1195 mutation, it is to be understood that the cancer may also be characterised by other mutations (including those not relating to MET). Similarly, a MET protein comprising a MET 1092, MET 1094 or MET 1195 mutation may also have other mutations at other amino acid positions.

It is also to be understood that a cancer that is characterised by a MET 1092, MET 1094 or MET 1195 mutation may be characterised by one or more of the specified mutations, e.g. a MET 1092 and a MET 1094 mutation, a MET 1092 and a MET 1195 mutation, a MET 1094 and a MET 1195 mutation, or a MET 1092, MET 1094 and MET 1195 mutation. Similarly, a MET protein comprising a MET 1092, MET 1094 or MET 1195 mutation may comprise one or more of the specified mutations.

In any embodiment where a cancer that is characterised by a MET 1092, MET 1094 or MET 1195 mutation is mentioned, the cancer may be characterised by a MET 1092 and a MET 1094 mutation.

In any embodiment where a cancer that is characterised by a MET 1092, MET 1094 or MET 1195 mutation is mentioned, the cancer may be characterised by a MET 1092 and a MET 1195 mutation.

In any embodiment where a cancer that is characterised by a MET 1092, MET 1094 or MET 1195 mutation is mentioned, the cancer may be characterised by a MET 1094 and a MET 1195 mutation.

In any embodiment where a cancer that is characterised by a MET 1092, MET 1094 or MET 1195 mutation is mentioned, the cancer may be characterised by a MET 1092, a MET 1094 and a MET 1195 mutation.

In any embodiment where a MET mutation (for example a MET 1092, MET 1094 or MET 1195 mutation) is mentioned, the mutation may be an amino acid substitution.

An "amino acid substitution" may include in frame indels. An "in frame indel" is is an insertion and/or deletion of an amino acid that is in the reading frame and therefore resulting in either an extra amino acid or a lost amino acid at a given position.

In any embodiment where a MET mutation (for example a MET 1092, MET 1094 or MET 1195 mutation) is mentioned, the mutation may be an indel.

In any embodiment where a MET mutation (for example a MET 1092, MET 1094 or MET 1195 mutation) is mentioned, the mutation may be an in frame indel.

In any embodiment where a MET mutation (for example a MET 1092, MET 1094 or MET 1195 mutation) is mentioned, the mutation may be a somatic mutation.

In any embodiment where a MET mutation (for example a MET 1092, MET 1094 or MET 1195 mutation) is mentioned, the MET mutation may be a germ-line mutation.

In any embodiment where a MET mutation (for example a MET 1092, MET 1094 or MET 1195 mutation) is mentioned, the MET mutation may be a MET V1092 mutation. A MET V1092 mutation occurs when the valine amino acid at position 1092 of the wild-type protein is changed.

In any embodiment where a MET mutation (for example a MET 1092, MET 1094 or MET 1195 mutation)is mentioned, the MET mutation may be a MET V1092I mutation. A MET V1092I mutation occurs when the valine amino acid at position 1092 of the wild-type protein is substituted by an isoleucine amino acid.

In any embodiment where a MET mutation (for example a MET 1092, MET 1094 or MET 1195 mutation)is mentioned, the MET mutation may be a MET H1094 mutation. A MET H1094 mutation occurs when the histidine amino acid at position 1094 of the wild-type protein is changed.

In any embodiment where a MET mutation (for example a MET 1092, MET 1094 or MET 1195 mutation)is mentioned, the MET mutation may be a MET H1094L mutation. A MET H1094L mutation occurs when the histidine amino acid at position 1094 of the wild-type protein is substituted by a leucine amino acid.

In any embodiment where a MET mutation (for example a MET 1092, MET 1094 or MET 1195 mutation)is mentioned, the MET mutation may be a MET L1195 mutation. A MET L1195 mutation occurs when the leucine amino acid at position 1195 of the wild-type protein is changed.

In any embodiment where a MET mutation (for example a MET 1092, MET 1094 or MET 1195 mutation) is mentioned, the MET mutation may be a MET L1195F mutation. A MET L1195F mutation occurs when the leucine amino acid at position 1195 of the wild-type protein is substituted by a phenylalanine amino acid.

In one embodiment there is provided a c-Met inhibitor for use in the treatment of cancer, where the cancer is characterised by a MET VI0921, MET H1094L or MET L1195F mutation.

In one embodiment 2. of the disclosure there is provided a c-Met inhibitor for use in the treatment of cancer, where the cancer is characterised by a V1092I mutation.

In one embodiment of the disclosure there is provided a c-Met inhibitor for use in the treatment of cancer, where the cancer is characterised by a MET H1094L mutation.

In one embodiment of the invention there is provided a c-Met inhibitor for use in the treatment of cancer, where the cancer is characterised by a MET L1195F mutation.

As already mentioned, cancer cells characterised by a MET 1092, MET 1094 or MET 1195 mutation express MET protein comprising at least one of the stated variances. As such, the relevant cancer cells carry corresponding mutations in the nucleic acid sequences coding for the relevant mutant proteins. Such mutant nucleic acids can also be used as biomarkers for c-Met inhibition and therefore also form embodiments of the specification.

In one embodiment there is provided a c-Met inhibitor for use in the treatment of cancer, where the cancer is characterised by a MET_c.3583C>T mutation.

A "MET_c.3583C>T mutation" occurs when a cytosine nucleotide at position 3583 of the nucleic acid sequence encoding the wild-type MET protein is substituted by a thymine nucleotide. A MET_c.3583C>T mutation encodes for a MET protein comprising a MET L1195F mutation.

The nucleic acid sequence encoding the wild-type MET protein is described in RefSeq: NCBI Reference Sequence Database (RefSeq Accession NM_00245), which is provided herein (SEQ ID NO: 2). The provided RefSeq includes noncoding sequences ("untranslated regions" or "UTRs"), including a UTR sequence 202 nucleotides in length prior to the start of coding sequence.

RefSeq: NCBI Reference Sequence Database (RefSeq Accession NM_00245) is used as the reference sequence when describing the genetic mutations discussed in the present specification. For example, references to a nucleic acid with a MET_c.3583C>T mutation describe a nucleic acid where the cytosine nucleotide at position 3785 of the sequence recited herein has been replaced by a thymine nucleotide.

"Cancer" refers to the uncontrolled growth of cells resulting in tumours or growths in a patient's body. "Cancer" and "tumour" are used interchangeably to describe the physical growths present in a cancer patient's body. "Cancer" includes both non-metastatic cancer and metastatic cancer, such that treating cancer may comprise treatment of both primary tumours and also local or distant metastases.

Cancer tumours are heterogeneous in nature, and each tumour in a cancer patient's body may contain separate populations of cells which are proliferating in an uncontrolled fashion for different reasons. For example, a given tumour may contain different populations of cells whose growth is being driven by different aberrations in their cellular machinery, for example different DNA mutations. As such, when it is mentioned that a cancer is characterised by a MET 1092, MET 1094 or MET 1195 mutation, and therefore comprises cells that express MET protein comprising a MET 1092, MET 1094 or MET 1195 mutation, not all of the cancer cells may express such mutant MET proteins.

In any embodiment where cancer is mentioned, the cancer may be lung cancer (for example small cell lung cancer or non-small cell lung cancer), gastric cancer, brain cancer (for example glioblastoma), colorectal cancer, breast cancer (for example triple negative breast cancer) or renal cancer (for example renal clear cell carcinoma or papillary renal cell carcinoma).

In any embodiment where cancer is mentioned, the cancer may be renal cancer (for example renal clear cell carcinoma or papillary renal cell carcinoma).

In any embodiment where cancer is mentioned, the cancer may be papillary renal cell carcinoma.

In any embodiment where papillary renal cell carcinoma is mentioned, the papillary renal cell carcinoma may be Type I papillary renal cell carcinoma.

"Type I papillary renal cell carcinoma" is a histological subtype characterised by a lining of small cells with clear to basophilic cytoplasm.

In any embodiment where papillary renal cell carcinoma is mentioned, the papillary renal cell carcinoma may be Type II papillary renal cell carcinoma.

"Type II papillary renal cell carcinoma" is a histological subtype characterised by a lining of by large cells with abundant eosinophilic cytoplasm.

The skilled person is able to distinguish between histological subtypes of papillary renal cell carcinoma using the above and other histological criteria.

In one embodiment of the invention there is provided a c-Met inhibitor for use in the treatment of cancer, where the cancer is characterised by a 1195 mutation, and the c-Met inhibitor is savolitinib or a pharmaceutically acceptable salt thereof, which is administered in a pharmaceutical composition comprising 500 - 1000 mg of savolitinib in its free base form, dosed once daily.

In one embodiment of the invention there is provided a c-Met inhibitor for use in the treatment of cancer, where the cancer is characterised by a MET 1195 mutation, and the c-Met inhibitor is savolitinib or a pharmaceutically acceptable salt thereof, which is administered in a pharmaceutical composition comprising 600 mg of savolitinib in its free base form, dosed once daily.

In one embodiment of the invention there is provided a c-Met inhibitor for use in the treatment of cancer, where the cancer is characterised by a MET L1195F mutation, and the c-Met inhibitor is savolitinib or a pharmaceutically acceptable salt thereof, which is administered in a pharmaceutical composition comprising 600 mg of savolitinib in its free base form, dosed once daily.

In one embodiment there is provided a c-Met inhibitor for use in the treatment of cancer, where the cancer is papillary renal cell carcinoma that is characterised by a MET V1092I, MET H1094L or MET L1195F mutation.

In one embodiment of the invention there is provided a c-Met inhibitor for use in the treatment of cancer, where the cancer is papillary renal cell carcinoma that is characterised by a MET L1195F mutation, and the c-Met inhibitor is savolitinib or a pharmaceutically acceptable salt thereof.

In one embodiment of the invention there is provided a c-Met inhibitor for use in the treatment of cancer, where the cancer is papillary renal cell carcinoma that is characterised by a MET L1195F mutation, and the c-Met inhibitor is savolitinib or a pharmaceutically acceptable salt thereof, which is administered in a pharmaceutical composition comprising 600 mg of savolitinib in its free base form, dosed once daily.

In one embodiment of the invention there is provided a c-Met inhibitor for use in the treatment of cancer, where the cancer is Type II papillary renal cell carcinoma that is characterised by a MET L1195F mutation, and the c-Met inhibitor is savolitinib or a pharmaceutically acceptable salt thereof, which is administered in a pharmaceutical composition comprising 600 mg of savolitinib in its free base form, dosed once daily.

In one embodiment of the invention there is provided savolitinib for use in the treatment of Type II papillary renal cell carcinoma that is characterised by a MET L1195F mutation, where the savolitinib is administered in a pharmaceutical composition comprising 600 mg of savolitinib in its free base form, dosed once daily.

In one embodiment there is provided a method of screening cancer patients to establish their suitability for treatment with a c-Met inhibitor, comprising analysing a representative sample of a patient's cancer *in-vitro* to determine whether the patient's cancer is characterised by a MET 1092, MET 1094 or MET 1195 mutation, where if the patient's cancer is found to be characterised by a MET 1092, MET 1094 or MET 1195 mutation, the patient is suitable for treatment with a c-Met inhibitor.

In one embodiment there is provided a method of screening cancer patients to establish their suitability for treatment with a c-Met inhibitor, comprising obtaining a representative sample of a patient's cancer, analysing the sample *in-vitro* to determine whether the patient's cancer is characterised by a MET 1092, MET 1094 or MET 1195 mutation, where if the patient's cancer is found to be characterised by a MET 1092, MET 1094 or MET 1195 mutation, the patient is suitable for treatment with a c-Met inhibitor.

In one embodiment there is provided a method of determining whether a cancer patient is likely to benefit from treatment with a c-Met inhibitor, comprising *in-vitro* testing a representative sample of a patient's cancer for the presence of a MET 1092, MET 1094 or MET 1195 mutation to determine whether the patient's cancer is characterised by a MET 1092, MET 1094 or MET 1195 mutation, where if the patient's cancer is found to be characterised by a MET 1092, MET 1094 or MET 1195 mutation, the patient is more likely to benefit from treatment with a c-Met inhibitor.

In one embodiment there is provided a method for selecting a suitable treatment regime for a cancer patient, comprising the steps of:
a) determining the presence of a MET 1092, MET 1094 or MET 1195 mutation in a patient's cancer cells by analysing in a representative sample of a patient's cancer; and
b) selecting a treatment regime comprising the administration of a c-Met inhibitor if a MET L1195F mutation is present in a patient's cancer cells.

In one embodiment there is provided a method for selecting a suitable treatment regime for a cancer patient, comprising the steps of:
a) obtaining a representative sample of a patient's cancer;
b) determining the presence of a MET 1092, MET 1094 or MET 1195 mutation in a patient's cancer cells by analysing the sample; and
b) selecting a treatment regime comprising the administration of a c-Met inhibitor if a MET 1092, MET 1094 or MET 1195 mutation is present in a patient's cancer cells.

In one embodiment of the invention there is provided a method of screening cancer patients to establish their suitability for treatment with a c-Met inhibitor, comprising analysing a representative sample of a patient's cancer *in-vitro* to determine whether the patient's cancer is characterised by a 1195 mutation, where if the patient's cancer is found to be characterised by a MET 1195 mutation, the patient is suitable for treatment with a c-Met inhibitor, where the c-Met inhibitor is savolitinib or a pharmaceutically acceptable salt thereof, which is administered in a pharmaceutical composition comprising 500-1000 mg of savolitinib in its free base form, dosed once daily.

In one embodiment of the invention there is provided a method of screening cancer patients to establish their suitability for treatment with a c-Met inhibitor, comprising analysing a representative sample of a patient's cancer *in-vitro* to determine whether the patient's cancer is characterised by a MET 1195 mutation, where if the patient's cancer is found to be characterised by a MET 1195 mutation, the patient is suitable for treatment with a c-Met inhibitor, where the c-Met inhibitor is savolitinib or a pharmaceutically acceptable salt thereof, which is administered in a pharmaceutical composition comprising 600 mg of savolitinib in its free base form, dosed once daily.

In one embodiment of the invention there is provided a method of screening cancer patients to establish their suitability for treatment with a c-Met inhibitor, comprising analysing a representative sample of a patient's cancer *in-vitro* to determine whether the patient's cancer is characterised by a MET L1195F mutation, where if the patient's cancer is found to be characterised by a MET L1195F, the patient is suitable for treatment with a c-Met inhibitor, where the c-Met inhibitor is savolitinib or a pharmaceutically acceptable salt thereof, which is administered in a pharmaceutical composition comprising 600 mg of savolitinib in its free base form, dosed once daily.

In one embodiment there is provided a method of screening cancer patients to establish their suitability for treatment with a c-Met inhibitor, comprising analysing a representative sample of a patient's cancer *in-vitro* to determine whether the patient's cancer is characterised by a MET VI0921, MET H1094L or MET L1195F mutation, where if the patient's cancer is found to be characterised by a MET V1092I, MET H1094L or MET L1195F, the patient is suitable for treatment with a c-Met inhibitor, where the cancer is papillary renal cell carcinoma.

In one embodiment of the invention there is provided a method of screening cancer patients to establish their suitability for treatment with a c-Met inhibitor, comprising analysing a representative sample of a patient's cancer *in-vitro* to determine whether the patient's cancer is characterised by a MET L1195F mutation, where if the patient's cancer is found to be characterised by a MET L1195F, the patient is suitable for treatment with a c-Met inhibitor, where the cancer is papillary renal cell carcinoma, and the c-Met inhibitor is savolitinib or a pharmaceutically acceptable salt thereof.

In one embodiment of the invention there is provided a method of screening cancer patients to establish their suitability for treatment with a c-Met inhibitor, comprising analysing a representative sample of a patient's cancer *in-vitro* to determine whether the patient's cancer is characterised by a MET L1195F mutation, where if the patient's cancer is found to be characterised by a MET L1195F, the patient is suitable for treatment with a c-Met inhibitor, where the cancer is papillary renal cell carcinoma, and the c-Met inhibitor is savolitinib or a pharmaceutically acceptable salt thereof, which is administered in a pharmaceutical composition comprising 600 mg of savolitinib in its free base form, dosed once daily.

In one embodiment of the invention there is provided a method of screening cancer patients to establish their suitability for treatment with a c-Met inhibitor, comprising analysing a representative sample of a patient's cancer *in-vitro* to determine whether the patient's cancer is characterised by a MET L1195F mutation, where if the patient's cancer is found to be characterised by a MET L1195F, the patient is suitable for treatment with a c-Met inhibitor, where the cancer is type II papillary renal cell carcinoma, which is administered in a pharmaceutical composition comprising 600 mg of savolitinib in its free base form, dosed once daily.

In one embodiment of the invention there is provided a method of screening papillary renal cell carcinoma patients to establish their suitability for treatment with savolitinib or a pharmaceutically acceptable salt thereof, comprising analysing a representative sample of a patient's cancer *in-vitro* to determine whether the patient's cancer is characterised by a mutation, where if the patient's cancer is found to be characterised by a MET L1195F mutation, the patient is suitable for treatment with with savolitinib or a pharmaceutically acceptable salt thereof.

In one embodiment there is provided the use of a c-Met inhibitor in the preparation of a medicament for the treatment of cancer, where the cancer is characterised by a MET 1092, MET 1094 or MET 1195 mutation.

In one embodiment there is provided a method for treating cancer in a patient whose cancer is characterised by a MET 1092, MET 1094 or MET 1195 mutation, which comprises administering to said patient a therapeutically effective amount of a c-Met inhibitor.

In one embodiment there is provided a method for treating cancer in a patient in need of such treatment, comprising the steps of:
a) determining whether the patient's cancer is characterised by a MET 1092, MET 1094 or MET 1195 mutation; and
b) administering a therapeutically effective amount of a c-Met inhibitor to the patient if the patient's cancer is found to be characterised by a MET 1092, MET 1094 or MET 1195 mutation.

In one embodiment there is provided a method for treating cancer in a patient in need of such treatment, comprising the steps of:
a) requesting a test whose results can determine whether the patient's cancer is characterised by a MET 1092, MET 1094 or MET 1195 mutation; and
b) administering a therapeutically effective amount of a c-Met inhibitor to the patient if the patient's cancer is found to be characterised by a MET 1092, MET 1094 or MET 1195 mutation.

In one embodiment there is provided a method for treating cancer in a patient in need of such treatment, comprising the steps of:
a) requesting a test whose results can determine whether the patient's cancer is characterised by a MET 1092, MET 1094 or MET 1195 mutation; and
b) prescribing a therapeutically effective amount of a c-Met inhibitor to the patient if the patient's cancer is found to be characterised by a MET 1092, MET 1094 or MET 1195 mutation.

In one embodiment there is provided a diagnostic kit suitable for the determination of a MET mutation (for example a MET 1092, MET 1094 or MET 1195 mutation), comprising degenerate primers suitable to amplify a target nucleic acid in the MET gene and optional instructions comprising amplification protocol and result analysis.

In any embodiment where a diagnostic kit suitable for the determination of a MET mutation is mentioned, the kit may comprise instructions comprising amplification protocol and result analysis.

In any embodiment where a diagnostic kit suitable for the determination of a MET mutation is mentioned, the kit may comprise buffers, enzymes, and containers for performing the amplification and analysis of the amplification products.

In any embodiment where a diagnostic kit suitable for the determination of a MET mutation is mentioned, the kit may comprise one or more DNA microarrays.

In any embodiment where a diagnostic kit suitable for the determination of a MET mutation is mentioned, the kit may comprise one or more control templates. Suitable control templates include nucleic acids isolated from normal tissue sample, and samples representing different variances in the reference genes.

In any embodiment where a diagnostic kit suitable for the determination of a MET mutation is mentioned, the kit may comprise two or more primer pairs, each pair capable of amplifying a different region of the MET gene (each region a site of potential variance) thereby providing a kit for analysis of the presence of several gene variances in a biological sample in one reaction or several parallel reactions.

Suitable primers may be labelled (for example fluorescently) to facilitate detection of the amplification products and consequent analysis of the nucleic acid variances. The kit may allow for more than one variance to be detected in one analysis. A combination kit will therefore comprise primers capable of amplifying different segments of the reference gene. The primers may be differentially labelled, for example using different fluorescent labels, so as to differentiate between the variances.

In any embodiment where a diagnostic kit suitable for the determination of a MET mutation is mentioned, the kit may allow for mutant forms of MET to be determined, wherein the mutation is present at any position across the gene.

In any embodiment where a diagnostic kit suitable for the determination of a MET mutation is mentioned, the kit allows for a MET 1092, MET 1094 or MET 1195 mutation to be determined.

In any embodiment where a diagnostic kit suitable for the determination of a MET mutation is mentioned, the kit allows for a MET V1092I, MET H1094L or MET L1195F mutation to be determined.

Determining whether or not a cancer is characterised by a certain mutation (for example whether or not the cancer comprises cells expressing MET protein comprising a mutation at positions 1092, 1094 or 1195 relative to wild-type MET protein) may be based on the analysis of the cell's genomic DNA, or by analysis of DNA transcripts or proteins encoded by the DNA. It may be desirable to confirm the presence of a given mutation in a cancer cell's genomic DNA by parallel analysis of transcripts and/or proteins in order to ensure that the mutation is indeed expressed in the patient's cancer cells.

There are a large number of analytical procedures well known in the art which may be used to detect mutations at one or more positions in a gene, which then code for corresponding mutations in a protein, such as the MET protein. In general, such detection methods require a mutation detection technique, optionally an amplification method (for example a polymerase chain reaction, or "PCR"), optionally a capture or enrichment method (for example hybridization capture in next generation sequencing methods) and optionally a signal generation system. Example methods for the detection of genetic mutations are discussed in Nollau et al. Clin. Chem. 1997, 43, 1114-1120; Anderson, S.M; Expert Rev. Mol. Diagn. 2011, 11, 635-642; Meyerson M. et al., Nat. Rev. Genet. 2010, 11, 685-696; and in standard textbooks, for example Laboratory Protocols for Mutation Detection, 2nd Edition; Edited by U. Landegren; Oxford University Press 1996 and PCR by Newton & Graham, BIOS Scientific Publishers Limited 1997.

In any embodiment where it is necessary to determine whether a cancer is characterised by a MET mutation (for example a MET 1092, MET 1094 or MET 1195 mutation), the determination may comprise a mutation detection technique.

In any embodiment where it is necessary to determine whether a cancer is characterised by a mutation (for example a MET 1092, MET 1094 or MET 1195 mutation), the determination may comprise a mutation detection technique and an amplification method.

In any embodiment where it is necessary to determine whether a cancer is characterised by a mutation (for example a MET 1092, MET 1094 or MET 1195 mutation), the determination may comprise a mutation detection technique and a capture or enrichment method.

In any embodiment where it is necessary to determine whether a cancer is characterised by a mutation (for example a MET 1092, MET 1094 or MET 1195 mutation), the determination may comprise a mutation detection technique and a signal generation system.

In any embodiment where it is necessary to determine whether a cancer is characterised by a mutation (for example a MET 1092, MET 1094 or MET 1195 mutation), the determination may comprise a mutation detection technique, an amplification method, a capture or enrichment method and a signal generation system.

A "mutation detection technique" can be performed by analysing DNA or RNA collected from biological samples by a variety of methods including PCR, hybridization with allele-specific probes, enzymatic mutation detection, chemical cleavage of mismatches, mass spectrometry or DNA sequencing (including minisequencing). Example mutation detection techniques include amplification refractory mutation system (ARMS™), amplification refractory mutation system linear extension (ALEX™), competitive oligonucleotide priming system (COPS), Taqman, Molecular Beacons, restriction fragment length polymorphism (RFLP), restriction site based PCR and fluorescence resonance energy transfer (FRET) techniques and oligonucleotide ligation assay (OLA).

Hybridization with allele specific probes can be conducted using: (1) allele specific oligonucleotides bound to a solid phase (e.g. glass, silicon, nylon membranes) which is contacted with the labelled sample in solution, for example as in many DNA chip applications; or, (2) bound sample (often cloned DNA or PCR amplified DNA) and labelled oligonucleotides in solution (either allele specific or short so as to allow sequencing by hybridization). Diagnostic tests may comprise a panel of mutations, often on a solid support, which enables the simultaneous determination of more than one mutation. Such hybridization probes are well known in the art (see e.g., Molecular Cloning: A Laboratory Manual, 4th Edition, Volumes 1-3; editors Green and Sambrook; Cold Spring Harbor, N.Y 2012, ISBN: 9781936113422) and may span two or more variance sites.

In any embodiment where a mutation detection technique is mentioned, the mutation detection technique may comprise contacting MET nucleic acid containing a putative mutation site with at least one nucleic acid probe.

In any embodiment where a mutation detection technique is mentioned, the mutation detection technique may comprise contacting MET nucleic acid containing a potential mutation site with at least one nucleic acid probe, where the probe hybridizes with a nucleic acid sequence including a mutation site containing complementary nucleotide bases at the mutation site under selective hybridization conditions.

Hybridization can be detected using labels known to one skilled in the art. Such labels include radioactive, fluorescent, dye, and enzymatic labels.

In any embodiment where a mutation detection technique is mentioned, the mutation detection technique may comprise contacting MET nucleic acid containing a potential mutation site with at least one nucleic acid primer.

In any embodiment where a mutation detection technique is mentioned, the mutation detection technique may comprise contacting MET nucleic acid containing a potential mutation site with at least one nucleic acid primer, where the primer preferentially hybridizes with a nucleic acid sequence including a mutation site containing complementary nucleotide bases at the variance site under selective hybridization conditions.

Oligonucleotides used as primers for specific amplification may carry the complementary nucleotide base to the mutation of interest in the centre of the molecule, so that amplification depends on differential hybridization (see e.g., Gibbs, et al. Nucl. Acids Res. 1989, 17, 2437) or at the extreme 3'-terminus of one primer where, under appropriate conditions, mismatch can prevent, or reduce polymerase extension (see, e.g., Prossner 1993, Tibtech, 11 238).

In any embodiment where a mutation detection technique is mentioned, the mutation detection technique may comprise sequencing at least one nucleic acid sequence and comparing the obtained sequence with the known wild type nucleic acid sequence.

In any embodiment where a mutation detection technique is mentioned, the mutation detection technique may comprise mass spectrometric determination of at least one nucleic acid sequence.

In any embodiment where a mutation detection technique is mentioned, the mutation detection technique may comprise performing a PCR.

Mutations in genomic nucleic acid may also be advantageously detected by techniques based on mobility shift in amplified nucleic acid fragments. For instance, Chen et al., Anal Biochem. 1996, 239, 61, describe the detection of single-base mutations by a competitive mobility shift assay. Moreover, assays based on the technique of Marcelino et al., BioTechniques 1999, 26, 1134-1148 are available commercially.

In any embodiment where a mutation detection technique is mentioned, the mutation detection technique may comprise using capillary heteroduplex analysis to detect the presence of mutations based on mobility shift of duplex nucleic acids in capillary systems as a result of the presence of mismatches.

An "amplification method" may be used to generate further amounts of nucleic acids detected during a mutation detection technique, making the subsequent analysis steps easier. Most amplification methods rely on an enzymatic chain reaction (such as a PCR, a ligase chain reaction, or a self-sustained sequence replication) or from the replication of all or part of the vector into which it has been cloned.

Many target and signal amplification methods have been described in the literature, for example, general reviews of these methods in Landegren, U. et al., Science 1988, 242, 229-237 and Lewis, R., Genetic Engineering News 1990, 10, 54-55. These amplification methods can be used in the methods described herein, and include PCR, PCR *in situ,* ligase amplification method (LAR), ligase hybridisation, Qβ bacteriophage replicase, transcription-based amplification system (TAS), genomic amplification with transcript sequencing (GAWTS), nucleic acid sequence-based amplification (NASBA) and *in-situ* hybridisation. Primers suitable for use in various amplification techniques can be prepared according to methods known in the art.

PCR is a nucleic acid amplification method described inter alia in U.S. Pat. Nos. 4,683,195 and 4,683,202. PCR consists of repeated cycles of DNA polymerase generated primer extension reactions. The target DNA is heat denatured and two oligonucleotides, which bracket the target sequence on opposite strands of the DNA to be amplified, are hybridised. These oligonucleotides become primers for use with DNA polymerase. The DNA is copied by primer extension to make a second copy of both strands. By repeating the cycle of heat denaturation, primer hybridisation and extension, the target DNA can be amplified a million fold or more in about two to four hours. PCR is a molecular biology tool, which must be used in conjunction with a detection technique to determine the results of amplification. An advantage of PCR is that it increases sensitivity by amplifying the amount of target DNA by 1 million to 1 billion fold in approximately 4 hours. PCR can be used to amplify any known nucleic acid in a diagnostic context (Mok et al., Gynaecologic Oncology, 1994, 52: 247-252,).

An allele specific amplification technique such as Amplification Refractory Mutation System (ARMS™) (Newton et al., Nucleic Acids Res., 1989, 17, 2503-2516) can also be used to detect single base mutations. Under the appropriate PCR amplification conditions a single base mismatch located at the 3'-end of the primer is sufficient for preferential amplification of the perfectly matched allele (Newton *et al.,* 1989, supra), allowing the discrimination of closely related species. The basis of an amplification system using the primers described above is that oligonucleotides with a mismatched 3'-residue will not function as primers in the PCR under appropriate conditions. This amplification system allows genotyping solely by inspection of reaction mixtures after agarose gel electrophoresis.

In any embodiment where an amplification method is mentioned, the amplification method may comprise a PCR.

Various "signal generation systems" can be used to analyse amplification products. Signal generation systems can be performed using any method capable of separating the amplification products according to their size, including automated and manual gel electrophoresis, mass spectrometry, and the like.

The methods of nucleic acid isolation, amplification and analysis are routine for one skilled in the art and examples of protocols can be found, for example, Molecular Cloning: A Laboratory Manual, 4th Edition, Volumes 1-3; editors Green and Sambrook; Cold Spring Harbor, N.Y 2012, ISBN: 9781936113422). A particularly useful protocol source for methods used in PCR amplification is PCR (Basics: From Background to Bench) by M. J. McPherson, S. G. Mailer, R. Beynon, C. Howe, Springer Verlag; 1st edition (October 15, 2000), ISBN: 0387916008,

In any embodiment where a MET mutation (for example a MET 1092, MET 1094 or MET 1195 mutation) is mentioned, the detection of a MET mutation may comprise a signal generation systems which is gel electrophoresis.

In any embodiment where a MET mutation (for example a MET 1092, MET 1094 or MET 1195 mutation) is mentioned, the detection of a MET mutation may comprise a signal generation systems which is mass spectrometry.

Methods that may be used to detect the protein mutations described herein include mass spectrometry or antibody based approaches that are designed to detect the amino acid substitution at the indicated positions.

A "representative sample of a patient's cancer" can be any tumour tissue or tumour-cell containing sample obtained or obtainable from a cancer patient, including any sample containing tumour nucleic acid, such as circulating free DNA (ctDNA). Samples of a patient's cancer may include a blood, mouth swab, biopsy, or other body fluid or tissue obtained from a patient. Particular examples include circulating tumour cells or circulating tumour DNA present in a patient's plasma or serum, cells isolated from the ascites fluid of ovarian cancer patients, lung sputum obtained from patients with tumours within the lung, a fine needle aspirate obtained from a breast cancer patient, urine, peripheral blood, a cell scraping, a hair follicle, a skin punch or a buccal sample.

Representative samples of a patient's cancer for mutation status testing may equally be nucleic acid sequences corresponding to the sequence obtained in a representative sample of a patient's cancer, that is to say that all or a part of the region in the sample nucleic acid may firstly be amplified using any convenient technique e.g. polymerase chain reaction (PCR), before analysis. The nucleic acid may be genomic DNA or fractionated or whole cell RNA. In some embodiments the RNA is whole cell RNA and is used directly as the template for labelling a first strand cDNA using random primers or poly A primers. The nucleic acid or protein in a sample of a patient's cancer may be extracted from the sample according to standard methodologies, e.g. those referenced in Molecular Cloning: A Laboratory Manual, 4th Edition, Volumes 1-3; editors Green and Sambrook; Cold Spring Harbor, N.Y 2012, ISBN: 9781936113422,

A representative sample of a patient's cancer may previously have been taken from the patient. Such samples may be preserved by freezing or fixed and embedded in formalin-paraffin or other media. Alternatively, a fresh sample may be obtained and used.

In any embodiment where a representative sample of a patient's cancer is mentioned, the representative sample of a patient's cancer may comprise a tumour cell sample.

In any embodiment where a representative sample of a patient's cancer is mentioned, the representative sample of a patient's cancer may comprise a sample containing tumour nucleic acid.

In any embodiment where a representative sample of a patient's cancer is mentioned, the representative sample of a patient's cancer may comprise a sample containing tumour DNA.

In any embodiment where a representative sample of a patient's cancer is mentioned, the representative sample of a patient's cancer may comprise a sample containing tumour circulating free DNA.

In embodiment [A] there is provided a c-Met inhibitor for use in the treatment of cancer, where the cancer is characterised by a MET 1092, MET 1094 or MET 1195 mutation.

In embodiment [B] there is provided a c-Met inhibitor for use in the treatment of cancer as described in embodiment [A], where the MET 1092, MET 1094 or MET 1195 mutation is an amino acid substitution.

In embodiment [C] there is provided a c-Met inhibitor for use in the treatment of cancer as described in embodiment [A] or embodiment [B], where the cancer is characterised by a MET V1092I, MET H1094L or MET L1195F mutation.

In embodiment [D] there is provided a c-Met inhibitor for use in the treatment of cancer as described in embodiment [C], where the cancer is characterised by a MET V1092I mutation.

In embodiment [E] there is provided a c-Met inhibitor for use in the treatment of cancer as described in embodiment [C], where the cancer is characterised by a MET H1094L mutation.

In embodiment [F] there is provided a c-Met inhibitor for use in the treatment of cancer as described in embodiment [A], where the cancer is characterised by a MET L1195F mutation.

In embodiment [G] there is provided a c-Met inhibitor for use in the treatment of cancer as described in any one of embodiments [A] to [F], where the cancer is lung cancer, gastric cancer or papillary renal cell carcinoma.

In embodiment [H] there is provided a c-Met inhibitor for use in the treatment of cancer as described in embodiment [G], where the cancer is papillary renal cell carcinoma.

In embodiment [I] there is provided a c-Met inhibitor for use in the treatment of cancer as described in embodiment [H], where the cancer is Type IIpapillary renal cell carcinoma.

In embodiment [J] there is provided a c-Met inhibitor for use in the treatment of cancer as described in any one of embodiments [A] to [I], where the c-Met inhibitor is savolitinib or a pharmaceutically acceptable salt thereof, crizotinib or a pharmaceutically acceptable salt thereof, tivantinib or a pharmaceutically acceptable salt thereof, cabozantinib or a pharmaceutically acceptable salt thereof, foretinib or a pharmaceutically acceptable salt thereof, capmatinib or a pharmaceutically acceptable salt thereof, onartuzumab, ficlatuzumab or rilotumumab.

In embodiment [K] there is provided a c-Met inhibitor for use in the treatment of cancer as described in embodiment [J], where the c-Met inhibitor is savolitinib or a pharmaceutically acceptable salt thereof.

In embodiment [L] there is provided a c-Met inhibitor for use in the treatment of cancer as described in any one of embodiments [A] to [K], where the treatment of cancer comprises analysing a representative sample of a patient's cancer *in-vitro* to determine whether the cancer is characterised by a MET 1092, MET 1094 or MET 1195 mutation, where if the patient's cancer is found to be characterised by a MET 1092, MET 1094 or MET 1195 mutation, the patient is suitable for treatment with a c-Met inhibitor.

In embodiment [M] there is provided a c-Met inhibitor for use in the treatment of cancer as described in embodiment [A], where the cancer is Type IIpapillary renal cell carcinoma that is characterised by a MET V1092I, MET H1094L or MET L1195F mutation, and the c-Met inhibitor is savolitinib or a pharmaceutically acceptable salt thereof, which is administered in a pharmaceutical composition comprising 600 mg of savolitinib in its free base form, dosed once daily.

In embodiment [N] there is provided a method of screening cancer patients to establish their suitability for treatment with a c-Met inhibitor, comprising analysing a representative sample of a patient's cancer *in-vitro* to determine whether the cancer is characterised by a MET 1092, MET 1094 or MET 1195 mutation, where if the patient's cancer is found to be characterised by a MET 1092, MET 1094 or MET 1195 mutation, the patient is suitable for treatment with a c-Met inhibitor.

In embodiment [O] there is provided the method of embodiment [N], comprising analysing a representative sample of a patient's cancer *in-vitro* to determine whether the cancer is characterised by a MET V1092I mutation, where if the patient's cancer is found to be characterised by a MET V1092I mutation, the patient is suitable for treatment with a c-Met inhibitor.

In embodiment [P] there is provided the method of embodiment [N], comprising analysing a representative sample of a patient's cancer *in-vitro* to determine whether the cancer is characterised by a MET H1 094L mutation, where if the patient's cancer is found to be characterised by a MET H1094L mutation, the patient is suitable for treatment with a c-Met inhibitor.

In embodiment [Q] there is provided the method of embodiment [N], comprising analysing a representative sample of a patient's cancer *in-vitro* to determine whether the cancer is characterised by a MET L1195F mutation, where if the patient's cancer is found to be characterised by a MET L1195F mutation, the patient is suitable for treatment with a c-Met inhibitor.

In embodiment [R] there is provided the method of any one of embodiments [N] to [Q], where the cancer is papillary renal cell carcinoma.

In embodiment [S] there is provided the method of any one of embodiments [N] to [R], where the c-Met inhibitor is savolitinib or a pharmaceutically acceptable salt thereof.

In embodiment [T] there is provided the use of a c-Met inhibitor in the preparation of a medicament for the treatment of cancer, where the cancer is characterised by a MET 1092, MET 1094 or MET 1195 mutation.

In embodiment [U] there is provided a method for treating cancer in a patient in need of such treatment, comprising the steps of:
a) requesting a test whose results can determine whether the patient's cancer is characterised by a MET 1092, MET 1094 or MET 1195 mutation; and
b) administering a therapeutically effective amount of a c-Met inhibitor to the patient if the patient's cancer is found to be characterised by a MET 1092, MET 1094 or MET 1195 mutation.

In embodiment [V] there is provided the method of embodiment [U], where the cancer is papillary renal cell carcinoma.

In embodiment [W] there is provided the method of embodiment [U] or embodiment [V], where the c-Met inhibitor is savolitinib or a pharmaceutically acceptable salt thereof.

According to the present invention, the c-Met inhibitor is savolitinib, or a pharmaceutically acceptable salt thereof, and the cancer is characterised by a MET 1195 mutation.

### EXAMPLES

The following tests support the embodiments described herein: a) phosphorylation of the MET receptor tyrosine kinase and effect of point mutations on sensitivity to MET inhibition with savolitinib; b) determination of the dose response of MET WT and MET point mutants to savolitinib and other MET inhibitors; c) transformation capacity of MET WT, MET mutant or parental Ba/F3 stable cell lines; d) MET L1195F mutant PRCC patient tumour response. During the description of the tests, generally:
i. The IC₅₀ value is the concentration of test compound that inhibits 50% of biological activity.
ii. Abbreviations used are explained in the main text or in the following paragraph, or would otherwise be well known to the skilled reader. aPTT = activated partial thromboplastin time; BCA = bicinchoninic acid; EDTA = ethylenediaminetetraacetic Acid; CTCAE = common terminology criteria for adverse events; DBP = diastolic blood pressure; DoR = duration of response; DVT = deep vein thrombosis; ECOG = Eastern Cooperative Oncology Group; ECG = electrocardiogram; FBS = Foetal Bovine Serum; h = Hour(s); GFR = glomerular filtration rate; HEK = Himan Embryonic Kidney cell line; IL-3 = interleukin-3; LDS = Loading Dye Solution; LMWH = low molecular weight heparin; NGS = next-generation sequencing; NYHA = New York heart association; ORR = overall response rate; OS = overall survival; PD = progressive disease; PBS = Phosphate buffered saline; PFS = progression-free survival; PK = pharmacokinetic; PO = by mouth; QD = once daily; RFP = red fluorescence protein; SC = sub-cutaneously; SBP = systolic blood pressure; TBST = tris-buffered saline - tween 20; ULN = upper limit of normal; WB =Western Blot.

### Test a): Phosphorylation of the MET receptor tyrosine kinase and effect of point mutations on sensitivity to MET inhibition with savolitinib

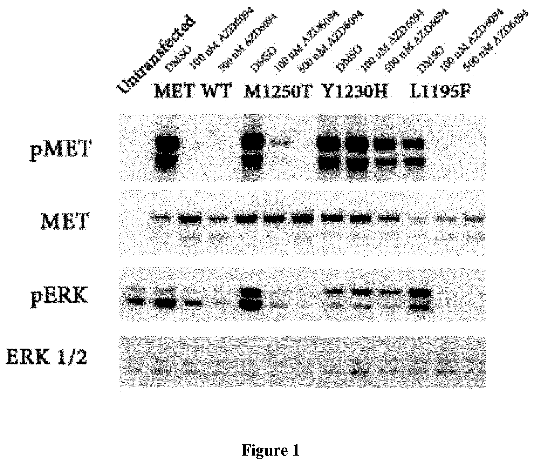

Transient transfections in HEK293T cells of MET WT and specific mutants with amino acid substitutions were investigated for their ability to activate the MET kinase using phosphorylated MET WB analysis, and downstream pathway activation with pERK1/2 WB analysis. MET WT and all MET mutants are activating when transiently over-expressed as demonstrated by DMSO treated condition and detection of phosphorylated MET (Y1234/Y1235). Novel MET mutant L1195F can activate MET and ERK1/2 and is sensitive to savolitinib inhibition to a similar extent as MET WT. Novel MET mutants V1092I and H1094L are also activated by over-expression and lead to downstream ERK pathway activation. In contrast, M1250T, a known robust activating MET mutant, is only partially inhibited at 100nM and Y1230H is completely resistant to savolitinib. The results of this test are shown in Figure 1 and demonstrate that MET L1195F, V1092I and H1094L are activating mutants of MET, and that downstream ERK1/2 signalling and can be inhibited with savolitinib treatment.

### Test b): Determination of the dose response of MET WT and MET point mutants to savolitinib and other MET inhibitors

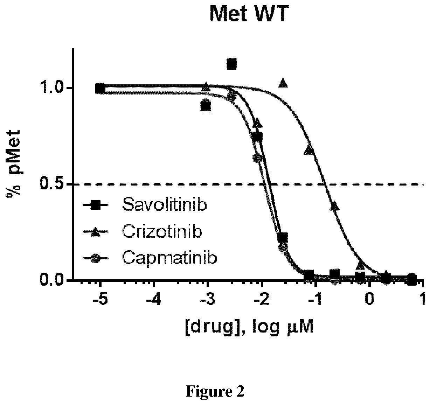

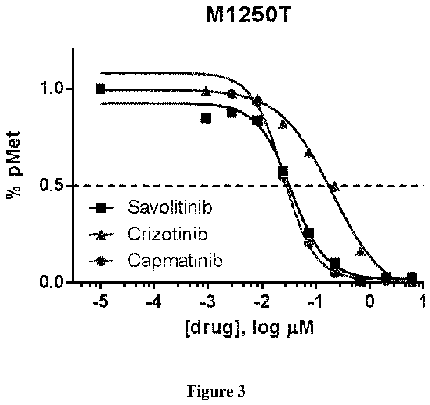

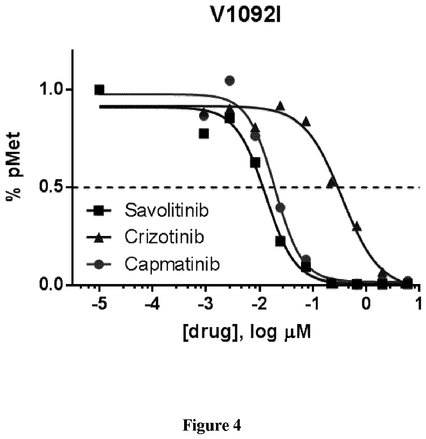

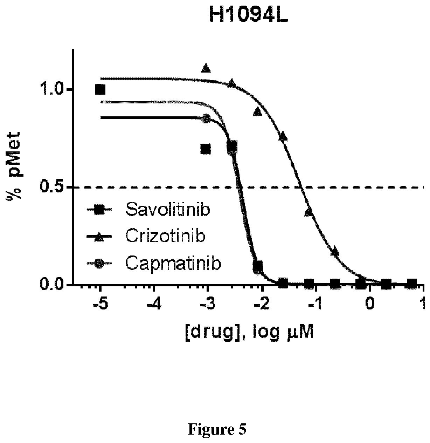

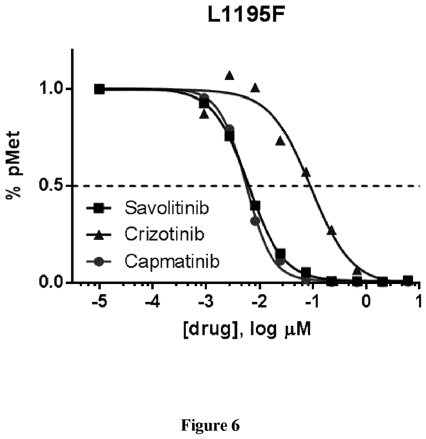

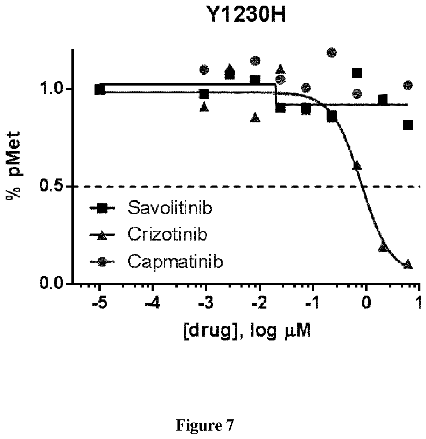

Transiently transfected HEK293T cells with MET WT and MET mutant constructs as indicated were treated with savolitinib, INC280 or crizotinib at indicated doses (range 2.7nM to 6.0µM) for 2.5 h to detect the extent of phospho-MET inhibition. The results are shown in Figures 2 - 7 and Table 1 below.

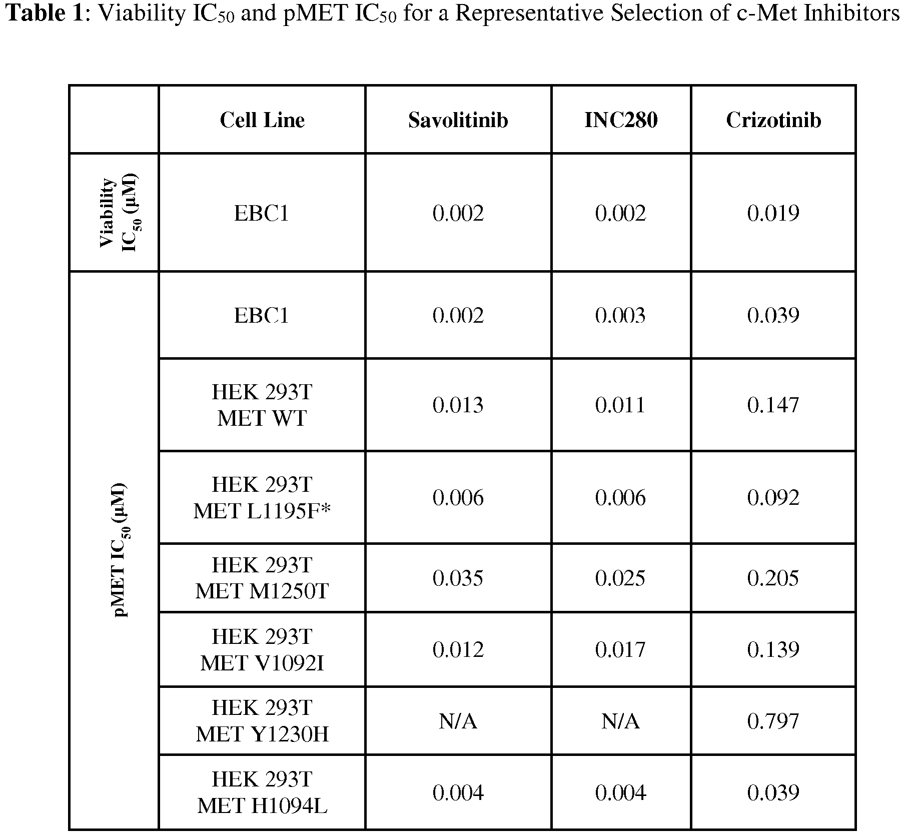

In Table 1 the IC₅₀ indicates the sensitivity of each MET WT and mutant protein toward inhibition with the relevant c-Met inhibitor. Savolitinib has an IC₅₀ of 13nM against WT MET protein, and 6nM against the known activating MET mutant M1250T IC₅₀. As such savolitinib has comparable IC₅₀ values toward both proteins. Similarly, cells that transiently express MET L1195F have a savolitinib IC₅₀ of 35nM also indicating that this MET mutant can be inhibited with savolitinib to a similar extent as for MET WT and the known activating M1250T mutant. Transient expression of MET V1092I and H1094L demonstrate that these mutant proteins are phosphorylated and that this phosphorylation can be inhibited with a MET inhibitor, such that the IC₅₀ of savolitinib is 12nM and 4nM respectively. In contrast, the MET Y1230H mutant is not sensitive to savolitinib inhibition since an IC₅₀ could not be determined due to the inability to inhibit at least 50% of the phospho-MET MSD signal. Phospho-MET MSD signal is normalized to total MET signal.

### Test c): Transformation capacity of MET WT, MET mutant or parental Ba/F3 stable cell lines

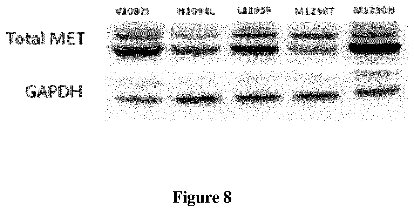

Stable cell line populations whose growth is dependent on IL-3 were assayed for their relative viability in the presence of IL-3 to establish the propensity for cell growth. IL-3 was removed to determine the transforming capability of each MET construct. Furthermore, HGF treatment conditions demonstrate the full contribution to MET dependant cell growth. The results of this test are shown in Figure 8.

Where parental Ba/F3 cells that are dependent on IL-3 for growth were not responsive to vector or HGF treatment, MET WT overexpression also did not confer a growth advantage in an IL-3 independent manner. In contrast, MET M1250T cells grow in the absence of IL-3 as expected and novel MET mutant L1195F is demonstrated to have the capacity to confer growth to a similar extent to MET M1250T. Other novel MET mutants (MET V1092I and MET H1094L, but not MET Y1230H) also have relatively greater capacity to confer IL-3 independent cell growth.

### Test d): MET L1195F mutant PRCC patient tumour response

A 56 year old woman was enrolled on a phase II trial to evaluate the efficacy of savolitinib (AZD6094/HMPL-504) in patients with PRCC (NCT02127710) in September 2014. A diagnostic sample was collected from the patient in December 2012 during resections of several metastatic lesions to the abdominal lymph nodes. This archival tumour sample was analysed by central pathology demonstrating high grade, poorly differentiated PRCC Type II. The patient started treatment with savolitinib on 9/2/14. Prior systemic therapies included suntinib and cytokines with best response being progressive disease (PD) for both prior lines of therapies. Savolitinib cycles used were 3 weeks.

Sequencing (NGS) of the diagnostic tumour sample (Foundation Medicine Inc, FMI) with a median exon coverage of 500x demonstrated the existence of a MET mutation MET_c.3583C>T with an allele fraction of 24%. The amino acid substitution of L1195F is located in exon 18 within the kinase domain of the MET receptor tyrosine kinase. This is reported by FMI as a variant of unknown significance. This tumour specimen harboured no co-occurring MET gene copy number variation, no other MET mutations and also did not co-occur in the background of Chromosome 7 gain which is a hallmark of PRCC and where both MET gene and it's ligand HGF gene loci reside. Chromosome 7 copy number was detected using an integrated copy number analysis of the 400 genes and 4200 SNPs measured in the FMI sequencing assay.

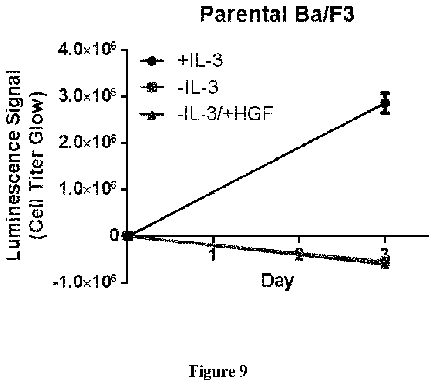

Subject 9203-002 had stable disease according to RECIST 1.1 criteria. However, this subject's best tumour response occurring at 36 weeks of savolitinib treatment was 29.7% decrease in the sum of the longest diameter of 4 target lesions (Figure 9). Sum of the target lesions are indicated with data labels in mm on the spider plot.

The following general experimental techniques were used during the above tests:

### MET WT and mutant clones

The synthetic gene pLVXIP_MET_FLAG was assembled from synthetic oligonucleotides and/or PCR products. The fragment was inserted into the vector backbone pLVXIRES-Puro_P839 cloning sites BamHI / EcoRI . The plasmid DNA was purified from transformed bacteria and concentration determined by UV spectroscopy. The final construct was verified by sequencing. The sequence (ABI) congruence within the insertion sites was 100%. Point mutations at indicated amino acid position (NM_000245) M1250T, Y1230H, L1195F, H1094L and V1092I were generated in the same manner as MET WT (Invitrogen by Thermo Fischer Scientific).

### Transient transfections and inhibitor treatments

Human Embryonic Kidney cell line (HEK293T) were transiently transfected with plasmids as outlined in MET WT and mutant clones section of M&M above. HEK293T cells were plated so 95% confluent on day of transfection (1 plate for each viral construct). The MET construct (24 µg) and Optimem (1.5 mL) were combined with Lipofectamine 2000 (60 µL per reaction, Invitrogen) in Optimem (1.5mL per reaction) for a MasterMix and incubated for 5 minutes at room temperature. Ad1.5 mL of Master Mix was added to each tube containing a construct for a final volume of ~3 mL. Each mixture was pipetted up and down to mix and let to sit for 20 minutes at room temperature. The plasmid mix (3 mL) was gently added to each 10 cm plate containing 12 mL of media and the plate incubated overnight. After 24 h, the media was removed and steps taken to gently pipette off 293T cells using sheer forces to re-plate at 1:10 in desired dosing plate (6 well or 96 well), which were let adhere overnight. Savolitinib (AZD6094) was diluted in DMSO at indicated concentrations and subsequently diluted into cell culture conditions for 2.5 h of treatment. Media was aspirated from cells and replaced with dosing media. The resultant plates were incubated for 2.5 h and immediately lysed in ice cold lysis buffer before being stored at -20°C.

### Cell lysate preparation

Lysis Buffer (TBS + 1% NP40) was supplemented with PhosSTOP phosphatase inhibitor and Complete Protease Inhibitor cocktails (Roche), 1 tablet each for every 10mL of lysis buffer. The mixture was kept cold. Cells were removed from wells by pipetting lysis buffer media (6-well dish) until semi-adherent 293T cells were suspended. These were then spun down at 1500 rpm for 5 min. Media was aspirated and the cell-pellet resuspended in 100µL of lysis buffer on ice. The suspension was immediately freeze/thawed twice in dry ice. Samples were then centrifuged at -14000 rpm for 10 minutes. Supernatant was transferred to a new tube (∼85µL), and kept on ice or frozen at -20°C until ready to use. A BCA assay was performed to determine protein concentration. Samples were normalized to 3.0 mg/ml in 90 µL using lysis buffer and 30µL LDS sample buffer and reducing agent solution added. The resultant suspension was centrifuged at 1500 RPM for 10 seconds, the samples heated to boiling (Thermo Cycler-200), centrifuged again, and allowed to cool.

### Western blots

40µg proteins from whole cell lysate were separated *via* gel electrophoresis using SDS-PAGE 10% Bis-Tris MidiGel (LifeTech). Following electrophoresis, separated proteins were transferred onto a nitrocellulose (LifeTech) membrane. Membranes were blocked to prevent nonspecific binding of antibodies with 5% non-fat dry milk in TBST. The membrane was then incubated with a primary antibody raised against the protein of interest and then a subsequent secondary antibody conjugated to HRP raised against the primary antibody. Finally, the membrane was incubated with a substrate detectable *via* enhanced chemiluminescence (ECL, Thermo Fisher Scientific) 3 min incubation at room temperature, and developed with imaging system (Fuji, ImageQuant LAS-4000).

Antibodies used included: MET (CST, 8198S, rabbit), pMET (CST, 3077S, rabbit), ERK 1/2 (CST, 4695S, rabbit), pERK (CST, 4370S, rabbit), secondary anti-Rabbit HRP conjugated (CST, 7074P2).

### MSD phosphoprotein assays

Whole cell lysates were prepared as above for use with the MULTI-SPOT Phospho(Tyr1349)/Total Met Assay (MesoScale Discovery, MSD). MSD provides a plate that has been pre-coated with capture antibodies for phosphorylated Met (Tyr1349) and total Met on spatially distinct spots. Added 150 µL of blocking solution into each well. Sealed the plate with an adhesive plate seal, and incubated for 1 h with vigorous shaking (300-1000 rpm) at room temperature. Washed the plate 3 times with 300 µL/well of Tris Wash Buffer. Added 25 µL of samples per well. Sealed the plate with an adhesive plate seal, and incubated for 1 h with vigorous shaking (300-1000 rpm) at room temperature. Washed the plate 3 times with 300 µL/well of Tris Wash Buffer. Added 25 µL of detection antibody solution to each well of the MSD plate - anti-total Met conjugated with an electrochemiluminescent compound, MSD SULFO-TAG label. Sealed the plate with an adhesive plate seal, and incubated for 1 h with vigorous shaking (300-1000 rpm) at room temperature. Washed the plate 3 times with 300 µL/well of Tris Wash Buffer. Added 150 µL of IX Read Buffer T to each well of the MSD plate. Loaded the plate into an MSD SECTOR® Imager for analysis. The SECTOR® Imager applies a voltage to the plate electrodes causes the labels bound to the electrode surface to emit light. The instrument measures intensity of emitted light to provide a quantitative measure of phosphorylated Met (Tyr1349) and total Met present in the sample. The percent phospho-Met is can be calculated using independent MSD phospho-Met and total Met protein with the MSD phospho-/total multiplex phosphoprotein assays.

### Stable cell line generation

IL-3 dependent murine pro B cell line, Ba/F3 were plated at 5.2 x 105 cells/mL density in 6-well plate with mutant viral constructs, polybrene at final concentration of 8 µg/mL, and filled to a 2 mL volume with complete media (RPMI-1640 (Gibco), 10% FBS, 1% L-glut, IL-3 1 ng/mL). Suspension culture was sealed with parafilm and centrifuged for 45 minutes at 1500-2000 rpm at 20°C then incubated overnight and replaced with complete media. At confluency (2.0 x 106 cells/mL, 2-4 days later, replaced media with adding selective pressure where media contains a final concentration of 0.5 µg/mL Puromycin. Maintained cells under selectin with Puromycin for 3 weeks. Cell populations were split and one well processed to verify infected clone in stable cell line. DNA was extracted using Qiagen Blood and Tissue Kit and DNA concentration verified with NanoDrop. DNA was amplified by PCR using Q5 Hot Start High Fidelity 2X Master Mix (New England BioLabs). PCR products were purified by electrophoresis on a 1% Agarose Gel with Ethidium Bromide. Band of the correct molecular weight was cut from the gel and purified using Wizard SV Gel Purification Kit (Promega). DNA concentration was verified with NanoDrop and 10nM purified DNA and 10µM Primers sequenced to confirm MET mutant clones stable cell line (Eton Bioscience). Sequences were confirmed with Sequencer 5.4. A negative control group of cells infected with polybrene and viral packages with no construct, and a positive control group of cells was infected with polybrene and a Red Fluorescence Protein (RFP) virus.

PCR Primers used include MET sense 143090519 and antisense 143090520. Sequencing Primers used include F1 143090521, F2 143090522, F3 143090523, R1 143090524, R2 143090525, R3 143090526. Sequences for the primers used to confirm mutant constructs at position 1195, 1092 and 1094 are represented below and in the sequence listings herein.

Met sequencing primers:

| | |
|---|---|
| F1 | AGATACGACGCCCGGGTGC (SEQ ID NO: 3) |
| F2 | AGAAAATCCACTGCGCCG (SEQ ID NO: 4) |
| F3 | CGTGCACAACAAGACAGG (SEQ ID NO: 5) |

Non-coding strand primers:

| | |
|---|---|
| R1 | TCTGGTCATCAGCTCCCA (SEQ ID NO: 6) |
| R2 | AGCAGGCTCAGCACGTTGG (SEQ ID NO: 7) |
| R3 | AACTGGTCCTCGGGGAAGG (SEQ ID NO: 8) |

For 1195 mutant constructs F2 and R3 primers were used. For 1092 mutant constructs F1 and R2 primers were used. For 1094 mutant constructs F1 and R2 primers were used.

### Cellular transformation assays

The cells were treated with complete media (+IL-3, a Ba/F3 dependant growth factor), media without IL-3 (-IL-3), and media without IL-3 but containing the ligand for MET, HGF (-IL-3/+HGF). Viability was measured with CellTiter-Glo® Cell Viability Assay (Promega) and 21 day live cell count was taken with Cellometer (Nexcelom). Parental Ba/F3 cell line was assayed as a negative control. All MET stable lines were assayed in duplicate in 6 well format. Briefly, cells were plated at 1.0 x106 cells/ml, culture under indicated conditions where either one of 3 conditions: + IL-3 (10 ng/mL), - IL-3, -IL-3/+HGF(100 ng/mL). CellTiter-Glo® Reagent directly to cells cultured in serum-supplemented medium at day 0 and 3 with a 1:1 volume of CellTiter-Glo® Reagent to cells, shake for 20 minutes at room temperature, transfer 100µl to a white opaque bottom plate, record with illuminometer (Tcan). Duplicate readings are averaged in each experiment (n=3).

### Tumour central histology

One 5µM thick section of the archival tumour specimen was used for H&E staining and central pathology review (Labcorp). Two pathologists were read the stained section to confirm the PRCC diagnosis and assign a histological subtype (Type I or Type2). Should oe pathologist be unsure of their analysis an external third pathologist with expertise in PRCC pathology read the case (Brigham and Woman's Hospital).

### Tumour sequencing

Methods and Materials are described elsewhere (Frampton et al, Nature Biotechnology 2013) Briefly, targeted NGS of a 400 gene panel (version T7) was conducted after DNA preparation and library construction in the Foundation Medicine Inc. CLIA laboratory. Proprietary analytics are used to call variants resulting from the FMI pipeline. Raw sequencing BAM files were re-analysed by AZ bioinformatic pipelines and confirm findings reported by FMI.

### Savolitinib clinical trial

MET L1195F mutant PRCC patient tumour response was determined during an open-label, single-arm, multicentre, global phase II trial to evaluate the efficacy of savolitinib (AZD6094 / HMPL-504) in patients with papillary renal cell carcinoma (PRCC) who are treatment naive or previously treated (ClinicalTrials.gov identifier: NCT02127710; web address: https://clinicaltrials.gov/ct2/show/NCT02127710). Key trial and dosing details that would allow the skilled clinical team to replicate the results are as follows:
*Primary Outcome Measures:* Assess the antitumor activity of AZD6094 in patients with PRCC and in the subgroup of MET-positive patients as measured by investigator-assessment of Overall Response Rate. The response rate is defined as the number (%) of subjects with at least one visit response of complete or partial response that is confirmed at least 4 weeks later.
*Secondary Outcome Measures:* PFS, change in target lesion tumour size from baseline, DoR and OS in patients with PRCC and in the subgroup of MET-positive patients. Progression-free survival is defined as the time from randomisation until the date of objective disease progression or death (by any cause in the absence of progression) regardless of whether the subject withdraws from randomised therapy or receives another anti-cancer therapy prior to progression.

Assess the safety and tolerability of AZD 6094. Safety profiles will be assessed in terms of AEs and laboratory data, vital signs and ECGs that will be collected for all patients.

PK of AZD6094 and major metabolites M2 and M3 for AUC, AUC(0-24), AUC(0-t), AUCss, Cmax, Css max, and Css min (Time Frame: Cycle 1 Day 8 and 15, Cycle 2 Day 1, Cycle 3, Day 1, and Cycle 4, Day 1). Characterise the PK of AZD6094 and the major metabolites M2 and M3 following administration to steady state after multiple dosing when given orally.

Assess change in target lesion tumour size from baseline. Change in Target Lesion is percentage change from baseline in tumour size at 12 weeks. This is based on RECIST target lesion measurements taken at baseline and at week 12. Target lesions are measureable tumour lesions.

Assess duration of response according to RECIST v1.1. Duration of Response is defined as the time from the date of first documented response until the date of documented progression or any cause death. In the case where a subject does not progress following response, the duration of response will be the same as the PFS censoring time.

Assess duration of overall survival according to RECIST v1.1. Overall Survival (OS) - Overall survival is defined as the time from the date of randomization until death due to any cause.

*Experimental:* AZD6094 600 mg daily continuously. All patients entering the study will take AZD6094 600 mg PO QD. Treatment will be given continuously. Drug: AZD6094. AZD6094 is a potent and selective small molecule c-Met kinase inhibitor. Other Name: HMPL-504.

*Detailed Description:* This is an open-label, single-arm, multicentre, global, Phase II, study designed to evaluate the efficacy and safety of AZD6094 in patients with PRCC who are treatment naive or previously treated.

An independent central pathology review of tumour samples will be used to confirm the diagnosis of PRCC of all patients enrolling. However, locally available pathology results confirming PRCC will be allowed for timely study entry.

All patients entering the study will take AZD6094 600 mg PO QD. Treatment will be given continuously.

*Eligibility:* Ages Eligible for Study: 18 Years to 99 Years (Adult, Senior); Genders Eligible for Study: Both; Accepts Healthy Volunteers: No.

*Inclusion criteria:* Provision of informed consent prior to any study specific procedures, sampling and analyses.

Histologically confirmed papillary renal cell carcinoma, which is locally advanced or metastatic.

Availability of an archival tumour sample or a pre-treatment fresh tumour sample for confirmation of PRCC by a central laboratory and other biomarker.

Treatment naive or have failed on previous treatment for PRCC. Previous treatments may include: targeted therapy (i.e. sunitinib, sorafenib, bevacizumab, pazopanib, temsirolimus, and everolimus), traditional immunotherapy (i.e. interferon-a, Interleukin-2), chemotherapy or a combination of chemoimmunotherapy.

ECOG performance status of 0 or 1.

At least one lesion, not previously irradiated, and not chosen for a biopsy if performed during the screening period that can be accurately measured at baseline and which is suitable for accurate repeated measurements.

Adequate haematological function defined as:
(ANC) ≥1500µL.
(Hgb) ≥9 g/dL.
Platelets ≥100,000/µL.

Adequate liver function defined as:
ALT and AST ≤2.5 x ULN.
Total bilirubin ≤1.5 x ULN.

Adequate renal function defined as GFR ≥ 40 mL/min,

Adequate coagulation parameters, defined as International Normalisation Ratio(INR) <1.5 x ULN or aPTT <1.5 x ULN.

Patients with known tumour thrombus or DVT are eligible if stable on LMWH for ≥4 weeks.

Females should be using adequate contraceptive measures should not be breast feeding, and must have a negative pregnancy test prior to start of dosing if of childbearing potential or must have evidence of non-childbearing potential.

Male patients should be willing to use barrier contraception, i.e. condoms.

Ability to swallow and retain oral medications.

Predicted life expectancy ≥12 weeks.

Aged at least 18 years.

Willingness and ability to comply with study and follow-up procedures.

Ability to understand the nature of this study and give written informed consent.

*Exclusion criteria*: Most recent chemotherapy, immunotherapy, chemoimmunotherapy, or investigational agents <21 days of the first dose of study treatment. Most recent targeted therapy <14 days of the first dose of study treatment.

Unresolved toxicities from any prior therapy greater than CTCAE Grade 1 at the time of starting study treatment with the exception of alopecia.

Prior or current treatment with a c-Met inhibitor.

Strong inducers or inhibitors of CYP3A4, strong inhibitors of CYP1A2, or CYP3A4 substrates with a narrow therapeutic range within 2 weeks before the first dose of study treatment (3 weeks for St John's Wort).

Wide field radiotherapy (including therapeutic radioisotopes such as strontium-89) administered ≤28 days or limited field radiation for palliation ≤7 days prior to starting study drug or has not recovered from side effects of such therapy.

Major surgical procedures ≤28 days of beginning study drug or minor surgical procedures ≤7 days. No waiting is required following port-a-cath placement.

Previously untreated brain metastases.

Current leptomeningeal metastases or spinal cord compression due to disease.

Acute or chronic liver or pancreatic disease.

Uncontrolled diabetes mellitus.

Gastrointestinal disease or other condition that will interfere significantly with the absorption, distribution, metabolism, or excretion of oral therapy.

Any of the following cardiac diseases currently or within the last 6 months:
Unstable angina pectoris.
Congestive heart failure (NYHA ≥ Grade 2.
Acute myocardial infarction.
Stroke or transient ischemic attack.

Inadequately controlled hypertension (i.e., SBP >160 mmHg or DBP >100 mmHg) (patients with values above these levels must have their blood pressure controlled with medication prior to starting treatment).

Mean resting correct QT interval (QTc) >470 msec obtained from triplicate ECGs.

Any clinically important abnormalities in rhythm, conduction or morphology of resting ECGs, e.g. complete left bundle branch block, third degree heart block, second degree heart block, PR interval >250 msec.

Any factors that increase the risk of QTc prolongation or risk of arrhythmic events such as heart failure, hypokalaemia, congenital or familial long QT syndrome or family history of unexplained sudden death under 40 years of age or any concomitant medications known to prolong QT interval.

Currently receiving treatment with therapeutic doses of warfarin sodium. LMWH is allowed.

Serious active infection at the time of treatment, or another serious underlying medical condition that would impair the ability of the patient to receive protocol treatment.

Known diagnosis of human immunodeficiency virus (HIV), hepatitis B, or hepatitis C.

Presence of other active cancers, or history of treatment for invasive cancer ≤5years. Patients with Stage I cancer who have received definitive local treatment at least 3 years previously, and are considered unlikely to recur are eligible. All patients with previously treated *in-situ* carcinoma (i.e., non-invasive) are eligible, as are patients with history of non-melanoma skin cancer.

Psychological, familial, sociological, or geographical conditions that do not permit compliance with the protocol.
## Claims

1. A c-Met inhibitor for use in the treatment of cancer, where the cancer is **characterised by** a MET 1195 mutation and where the c-Met inhibitor is savolitinib or a pharmaceutically acceptable salt thereof.

2. A c-Met inhibitor for use in the treatment of cancer as claimed in claim 1, where the MET 1195 mutation is an amino acid substitution.

3. A c-Met inhibitor for use in the treatment of cancer as claimed in claim 2, where the cancer is **characterised by** a MET L1195F mutation.

4. A c-Met inhibitor for use in the treatment of cancer as claimed in any one of claims 1 to 3, where the cancer is lung cancer, gastric cancer or papillary renal cell carcinoma.

5. A c-Met inhibitor for use in the treatment of cancer as claimed in claim 4, where the cancer is papillary renal cell carcinoma.

6. A c-Met inhibitor for use in the treatment of cancer as claimed in claim 5, where the cancer is Type II papillary renal cell carcinoma.

7. A c-Met inhibitor for use in the treatment of cancer as claimed in claim 1, where the cancer is Type II papillary renal cell carcinoma that is **characterised by** a MET L1195F mutation, and the savolitinib, or a pharmaceutically acceptable salt thereof, is administered in a pharmaceutical composition comprising 600 mg of savolitinib in its free base form, dosed once daily.

8. A method of screening cancer patients to establish their suitability for treatment with a c-Met inhibitor, comprising analysing a representative sample of a patient's cancer in-vitro to determine whether the cancer is **characterised by** a MET 1195 mutation, where if the patient's cancer is found to be **characterised by** a MET 1195 mutation, the patient is suitable for treatment with the c-Met inhibitor, and where the c-Met inhibitor is savolitinib or a pharmaceutically acceptable salt thereof.

9. The method claimed in claim 8, comprising analysing a representative sample of a patient's cancer in-vitro to determine whether the cancer is **characterised by** a MET L1195F mutation, where if the patient's cancer is found to be **characterised by** a MET L1195F mutation, the patient is suitable for treatment with the c-Met inhibitor, and where the c-Met inhibitor is savolitinib or a pharmaceutically acceptable salt thereof.

10. The method claimed in any one of claims 8 or 9, where the cancer is papillary renal cell carcinoma.

## Patentansprüche

1. c-Met-Inhibitor zur Verwendung bei der Behandlung von Krebs, wobei der Krebs durch eine MET-1195-Mutation gekennzeichnet ist und wobei es sich bei dem c-Met-Inhibitor um Savolitinib oder ein pharmazeutisch annehmbares Salz davon handelt.

2. c-Met-Inhibitor zur Verwendung bei der Behandlung von Krebs nach Anspruch 1, wobei es sich bei der MET-1195-Mutation um eine Aminosäuremutation handelt.

3. c-Met-Inhibitor zur Verwendung bei der Behandlung von Krebs nach Anspruch 2, wobei der Krebs durch eine MET-L1195F-Mutation gekennzeichnet ist.

4. c-Met-Inhibitor zur Verwendung bei der Behandlung von Krebs nach einem der Ansprüche 1 bis 3, wobei es sich bei dem Krebs um Lungenkrebs, Magenkrebs oder papilläres Nierenzellkarzinom handelt.

5. c-Met-Inhibitor zur Verwendung bei der Behandlung von Krebs nach Anspruch 4, wobei es sich bei dem Krebs um papilläres Nierenzellkarzinom handelt.

6. c-Met-Inhibitor zur Verwendung bei der Behandlung von Krebs nach Anspruch 5, wobei es sich bei dem Krebs um papilläres Nierenzellkarzinom Typ II handelt.

7. c-Met-Inhibitor zur Verwendung bei der Behandlung von Krebs nach Anspruch 1, wobei es sich bei dem Krebs um papilläres Nierenzellkarzinom Typ II, das durch eine MET-L1195F-Mutation gekennzeichnet ist, handelt und das Savolitinib oder ein pharmazeutisch annehmbares Salz davon in einer pharmazeutischen Zusammensetzung, die 600 mg Savolitinib in Form seiner freien Base umfasst, einmal täglich verabreicht wird.

8. Verfahren zum Screenen von Krebspatienten zur Ermittlung ihrer Eignung für eine Behandlung mit einem c-Met-Inhibitor, umfassend das Analysieren einer repräsentativen Probe einer Krebserkrankung eines Patienten in vitro, um zu bestimmen, ob der Krebs durch eine MET-1195-Mutation gekennzeichnet ist, wobei dann, wenn festgestellt wird, dass der Krebs des Patienten durch eine MET-1195-Mutation gekennzeichnet ist, der Patient für eine Behandlung mit einem c-Met-Inhibitor geeignet ist, und wobei es sich bei dem c-Met-Inhibitor um Savolitinib oder ein pharmazeutisch annehmbares Salz davon handelt.

9. Verfahren nach Anspruch 8, umfassend das Analysieren einer repräsentativen Probe einer Krebserkrankung eines Patienten in vitro, um zu bestimmen, ob der Krebs durch eine MET-L1195F-Mutation gekennzeichnet ist, wobei dann, wenn festgestellt wird, dass der Krebs des Patienten durch eine MET-L1195F-Mutation gekennzeichnet ist, der Patient für eine Behandlung mit einem c-Met-Inhibitor geeignet ist, und wobei es sich bei dem c-Met-Inhibitor um Savolitinib oder ein pharmazeutisch annehmbares Salz davon handelt.

10. Verfahren nach einem der Ansprüche 8 oder 9, wobei es sich bei dem Krebs um papilläres Nlerenzellkarzinom handelt.

## Revendications

1. Inhibiteur de c-Met destiné à être utilisé dans le traitement d'un cancer, où le cancer est **caractérisé par** une mutation MET 1195 et où l'inhibiteur de c-Met est le savolitinib ou un sel de celui-ci acceptable d'un point de vue pharmaceutique.

2. Inhibiteur de c-Met destiné à être utilisé dans le traitement d'un cancer tel que revendiqué dans la revendication 1, où la mutation MET 1195 est un remplacement d'acide aminé.

3. Inhibiteur de c-Met destiné à être utilisé dans le traitement d'un cancer tel que revendiqué dans la revendication 2, où le cancer est **caractérisé par** une mutation MET L1195F.

4. Inhibiteur de c-Met destiné à être utilisé dans le traitement d'un cancer tel que revendiqué dans l'une quelconque des revendications 1 à 3, où le cancer est un cancer du poumon, un cancer gastrique ou un carcinome papillaire du rein.

5. Inhibiteur de c-Met destiné à être utilisé dans le traitement d'un cancer tel que revendiqué dans la revendication 4, où le cancer est un carcinome papillaire du rein.

6. Inhibiteur de c-Met destiné à être utilisé dans le traitement d'un cancer tel que revendiqué dans la revendication 5, où le cancer est un carcinome papillaire du rein de type II.

7. Inhibiteur de c-Met destiné à être utilisé dans le traitement d'un cancer tel que revendiqué dans la revendication 1, où le cancer est un carcinome papillaire du rein de type II, qui est **caractérisé par** une mutation MET L1195F, et le savolitinib, ou un sel de celui-ci acceptable d'un point de vue pharmaceutique, est administré dans une composition pharmaceutique comprenant 600 mg de savolitinib sous sa forme de base libre, en une dose par jour.

8. Procédé de criblage de patients cancéreux afin d'établir leur aptitude à recevoir un traitement avec un inhibiteur de c-Met, comprenant l'étape d'analyse d'un échantillon représentatif d'un cancer d'un patient in vitro pour déterminer si le cancer est **caractérisé par** une mutation MET 1195, où, si l'on trouve que le cancer du patient est **caractérisé par** une mutation MET 1195, le patient est apte à recevoir un traitement avec l'inhibiteur de c-Met et où l'inhibiteur de c-Met est le savolitinib ou un sel de celui-ci acceptable d'un point de vue pharmaceutique.

9. Procédé revendiqué dans la revendication 8, comprenant l'étape d'analyse d'un échantillon représentatif d'un cancer d'un patient in vitro pour déterminer si le cancer est **caractérisé par** une mutation MET L1195F, où, si l'on trouve que le cancer du patient est **caractérisé par** une mutation MET L1195F, le patient est apte à recevoir un traitement avec l'inhibiteur de c-Met et où l'inhibiteur de c-Met est le savolitinib ou un sel de celui-ci acceptable d'un point de vue pharmaceutique.

10. Procédé revendiqué dans l'une quelconque des revendications 8 ou 9, où le cancer est un carcinome papillaire du rein.
